# EUROPEAN PATENT APPLICATION

(11) **EP 3 834 845 A1**
(43) Date of publication of application: **16.06.2021**
(21) Application number: 19846407.5
(22) Date of filing: 08.08.2019
(51) Int. Cl.: A61K 47/54, A61K 31/695, A61K 41/00, A61P 35/00, C07K 14/36, C07K 16/22, C07K 16/30, C07K 16/32, C07K 19/00, C09B 47/18, C12N 15/76

(54) **CONJUGATE OF BIOTIN VARIANT DIMER WITH PHTHALOCYANINE PIGMENT**

(30) Priority: 08.08.2018 JP 2018149295
(71) Applicant: The University of Tokyo, Bunkyo-ku, Tokyo 113-8654 (JP); Savid Therapeutics Inc., Tokyo 168-0064 (JP)
(72) Inventor: KANAI Motomu, Tokyo 113-8654 (JP); YAMATSUGU Kenzo, Tokyo 113-8654 (JP); TATSUMI Toshifumi, Tokyo 113-8654 (JP); TAKAHASHI Kazuki, Tokyo 113-8654 (JP); KODAMA Tatsuhiko, Tokyo 113-8654 (JP); TANAKA Toshiya, Tokyo 113-8654 (JP); YAMASHITA Takefumi, Tokyo 113-8654 (JP); SUGIYAMA Akira, Tokyo 113-8654 (JP); TSUKAGOSHI Masanobu, Tokyo 168-0064 (JP)
(74) Representative: Godemeyer Blum Lenze Patentanwälte Partnerschaft mbB - werkpatent
(86) International application number: PCT/JP2019/031336
(87) International publication number: WO 2020/032165

(57) **Abstract**

It is an object of the present invention to provide a conjugate of a biotin-modified dimer and a phthalocyanine dye, which is used in photoimmunotherapy. The present invention provides a conjugate of a compound represented by the following formula (1) or a salt thereof and a phthalocyanine dye: 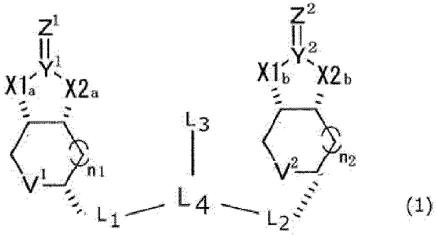 wherein the meaning of each of symbols is as defined in the specification.

## Description

### Technical Field

The present invention relates to a conjugate of a biotin-modified dimer and a phthalocyanine dye and the use thereof.

### Background Art

Avidin and biotin, or streptavidin and biotin have an extremely high affinity between them (Kd = 10⁻¹⁵ to 10⁻¹⁴ M). This is one of the strongest interactions between two biomolecules. At present, the interaction between avidin/streptavidin and biotin has been widely applied in the field of biochemistry, molecular biology, or medicine. A drug delivery method and a pretargeting method, in which high binding ability between avidin/streptavidin and biotin is combined with an antibody molecule, have been devised. In connection with these studies, a mutant streptavidin with a reduced affinity for natural biotin and a biotin-modified dimer having a high affinity for the mutant streptavidin with a low affinity for natural biotin are reported in Patent Document 1.

On the other hand, photoimmunotherapy is a therapeutic method of using a photosensitizer and an irradiation light to destroy specific cells in a body. When a photosensitizer is exposed to a light with a specific wavelength, it generates cytotoxic reactive oxygen species capable of inducing apoptosis, necrosis, and/or autophagy to around cells. For example, Patent Document 2 discloses a method of killing cells, comprising: a step of allowing cells comprising a cell surface protein to come into contact with a therapeutically effective amount of one or more antibodies-IR700 molecules, wherein the antibodies specifically bind to the cell surface protein; a step of irradiating the cells with a light at a wavelength of 660 to 740 nm and at a dose of at least 1 Jcm⁻²; and a step of allowing the cells to come into contact with one or more therapeutic agents at approximately 0 to 8 hours after the irradiation, thereby killing the cells. Patent Document 3 discloses a method of inducing cytotoxicity to a subject affected with a disease or a pathology, comprising: (a) administering to a subject, a therapeutically effective drug comprising a phthalocyanine dye such as IRDye (registered trademark) 700DX conjugated with a probe specifically binding to the cell of the subject; and (b) irradiating the cell with an appropriate excitation light in an amount effective for inducing cell death.

### Prior Art Documents

### Patent Documents

Patent Document 1: International Publication WO2015/125820
Patent Document 2: Japanese Patent No. 6127045
Patent Document 3: JP Patent Publication (Kohyo) No. 2017-524659 A

### Summary of Invention

### Object to be Solved by the Invention

It is an object of the present invention to provide a conjugate of a biotin-modified dimer and a phthalocyanine dye, which is used in photoimmunotherapy. It is another object of the present invention to provide a therapeutic kit, in which a combination of the above-described conjugate of a biotin-modified dimer and a phthalocyanine dye and a mutant streptavidin-molecular probe conjugate is used.

### Means for Solving the Object

As a result of intensive studies directed towards achieving the above-described objects, the present inventor has found that the proliferation of cancer cells can be suppressed by photoimmunotherapy using a conjugate of a biotin-modified dimer and a phthalocyanine dye, thereby completing the present invention.

Specifically, according to the present invention, the following inventions are provided.
[1] A conjugate of a compound represented by the following formula (1) or a salt thereof and a phthalocyanine dye: 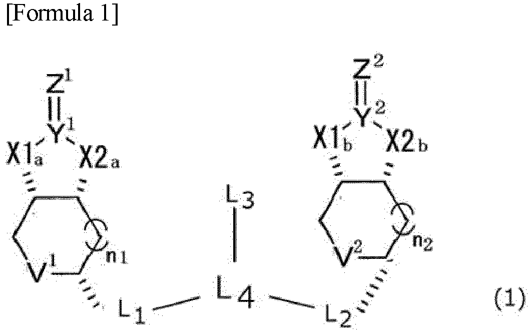 wherein
   X1a, X1b, X2a and X2b each independently represent O or NH,
   Y¹ and Y² each independently represent C or S,
   Z¹ and Z² each independently represent O, S or NH,
   V¹ and V² each independently represent S or S⁺-O⁻,
   n1 and n2 each independently represent an integer of 0 or 1,
   L₁ and L₂ each independently represent a divalent linking group,
   L₃ represents a group comprising a functional group capable of binding to a phthalocyanine dye at the terminus, and
   L₄ represents a trivalent linking group.
[2] The conjugate according to the above [1], wherein the compound represented by the above formula (1) is represented by the following formula (2): 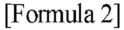 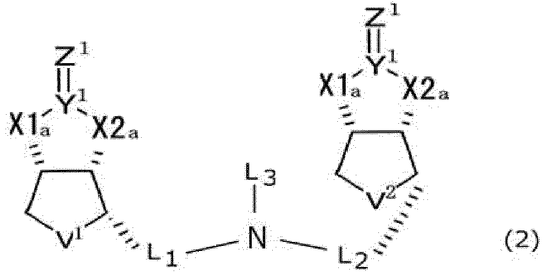 wherein each symbol is as defined in the above [1].
[3] The conjugate according to the above [1] or [2], wherein X1a, X1b, X2a and X2b represent NH; Y¹ and Y² represent C; Z¹ and Z² represent NH; and V¹ and V² represent S.
[4] The conjugate according to any one of the above [1] to [3], wherein L₁ and L₂ each independently represent a divalent linking group consisting of a combination of groups selected from -CONH-, -NHCO-, -COO-, -OCO-, -CO-, -O-, and an alkylene group containing 1 to 10 carbon atoms.
[5] The conjugate according to any one of the above [1] to [4], wherein L₃ represents a group consisting of a combination of groups selected from -CONH-, -NHCO-, -COO-, -OCO-, -CO-, -O-, and an alkylene group containing 1 to 10 carbon atoms, and further comprising an amino group at the terminus.
[6] The conjugate according to any one of the above [1] to [5], wherein the phthalocyanine dye is represented by the following formula (21): 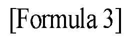 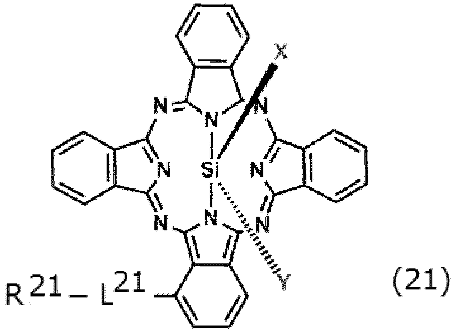 wherein L²¹ represents a divalent linking group; R²¹ represents a functional group capable of binding to the compound represented by the formula (1) or a salt thereof; and X and Y each independently represent a hydrophilic group, -OH, a hydrogen atom, or a substituent.
[7] The conjugate according to the above [6], wherein the hydrophilic group represented by X and/or Y is the following: 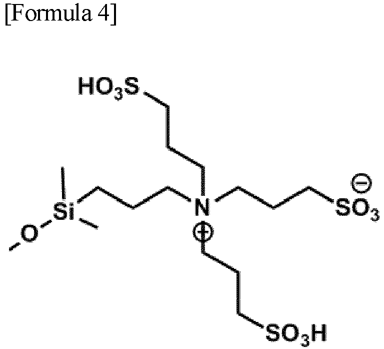
[8] The conjugate according to the above [6] or [7], wherein L²¹ represents a group consisting of a combination of groups selected from -CONH-, -NHCO-, -COO-, -OCO-, -CO-, -O-, and an alkylene group containing 1 to 10 carbon atoms.
[9] The conjugate according to any one of the above [6] to [8], wherein R²¹ represents an active ester group.
[10] A conjugate represented by any one of the following: 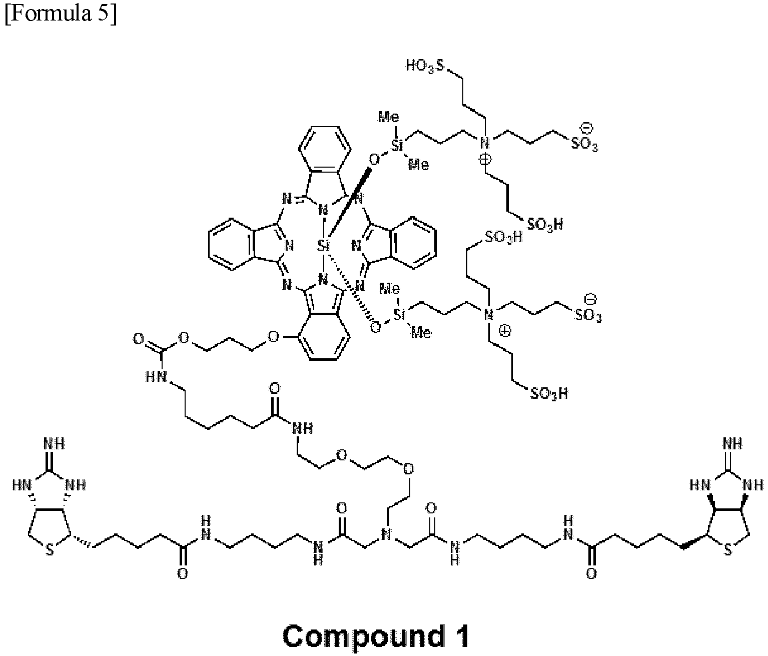 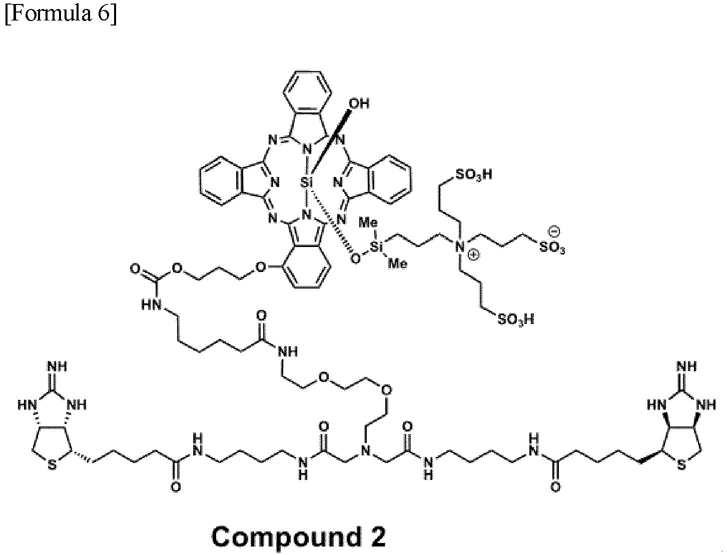 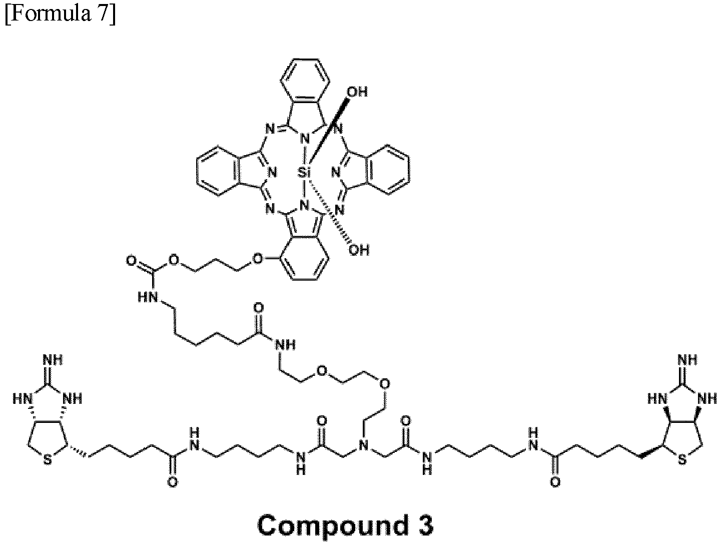
[11] A therapeutic agent comprising the conjugate according to any one of the above [1] to [10].
[12] A therapeutic kit, comprising: (1) the conjugate according to any one of the above [1] to [10]; and (b) a conjugate of a mutant streptavidin comprising the amino acid sequence as set forth in SEQ ID NO: 1 and a molecular probe.
[13] The therapeutic kit according to the above [12], wherein the molecular probe is an anti-EREG antibody, an anti-CEA antibody, or an anti-HER2 antibody.
[14] A fusion protein encoded by the nucleotide sequence as set forth in SEQ ID NO: 2, which can express a fusion protein of a mutant streptavidin comprising the amino acid sequence as set forth in SEQ ID NO: 1 and an scFv-type antibody that recognizes an EREG antigen.

### Advantageous Effects of Invention

The proliferation of cancer cells can be suppressed by photoimmunotherapy using the conjugate of a biotin-modified dimer and a phthalocyanine dye according to the present invention.

### Brief Description of Drawings

[Fig. 1] Fig. 1 shows the results of a cell proliferation suppression experiment using the conjugate of the present invention.
[Fig. 2] Fig. 2 shows the results of a cell proliferation suppression experiment using the conjugate of the present invention.
[Fig. 3] Fig. 3 shows an outline of a domain structure.
[Fig. 4] Fig. 4 shows a CBB-stained SDS-PAGE electrophoretic pattern of CEA-V2122.
[Fig. 5] Fig. 5 shows evaluation of the binding performance of CEA-Cupid with an antigen (CEACAM5).
[Fig. 6] Fig. 6 shows evaluation of the binding performance of CEA-Cupid with a modified biotin.
[Fig. 7] Fig. 7 shows stained cell images obtained using FITC-labeled CEA-V2122.
[Fig. 8] Fig. 8 shows time-series data of stained MKN45 cell images obtained using FITC-labeled CEA-V2122.
[Fig. 9] Fig. 9 shows photoactivatable compound-binding modified biotins.
[Fig. 10] Fig. 10 shows *in vitro* cytotoxicity obtained by a combination of CEA-Cupid and a photoactivatable compound-binding modified biotin.
[Fig. 11] Fig. 11 shows a change in the size of a tumor mass.
[Fig. 12] Fig. 12 shows *in vivo* cytotoxicity obtained by a combination of CEA-Cupid and a photoactivatable compound-binding modified biotin.
[Fig. 13] Fig. 13 shows histopathological images of *in vivo* cytotoxicity obtained by a combination of CEA-Cupid and a photoactivatable compound-binding modified biotin.
[Fig. 14] Fig. 14 shows the analysis of pathological sections using an anti-CEACAM5 antibody.
[Fig. 15] Fig. 15 shows a structural drawing of HER2-V2122.
[Fig. 16] Fig. 16 shows a CBB-stained SDS-PAGE electrophoretic pattern of HER2-V2122.
[Fig. 17] Fig. 17 shows evaluation of the binding performance of HER2-Cupid with an antigen (HER2).
[Fig. 18] Fig. 18 shows evaluation of the binding performance of HER2-Cupid with a modified biotin.
[Fig. 19] Fig. 19 shows *in vitro* cytotoxicity obtained by a combination of HER2-Cupid and a photoactivatable compound-binding modified biotin.

### Embodiment of Carrying out the Invention

Hereinafter, the present invention will be described in more detail.

### (1) Biotin-modified dimer

The present invention relates to a conjugate of a biotin-modified dimer and a phthalocyanine dye.

The biotin-modified dimer is a compound represented by the following formula (1) or a salt thereof, and is preferably a compound represented by the following formula (2) or a salt thereof. As such a biotin-modified dimer, the compound described in International Publication WO2015/125820 can be used.

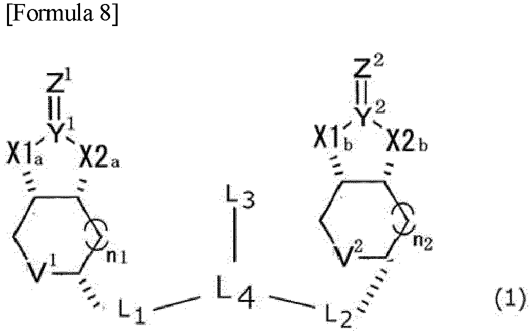

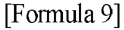

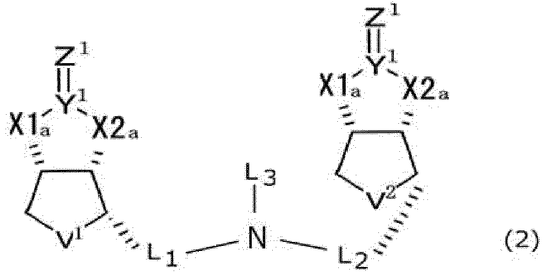

wherein
X1a, X1b, X2a and X2b each independently represent O or NH,
Y¹ and Y² each independently represent C or S,
Z¹ and Z² each independently represent O, S or NH,
V¹ and V² each independently represent S or S⁺-O⁻,
n1 and n2 each independently represent an integer of 0 or 1,
L₁ and L₂ each independently represent a divalent linking group,
L₃ represents a group comprising a functional group capable of binding to a phthalocyanine dye at the terminus, and
L₄ represents a trivalent linking group.

In the formula (1) and the formula (2), the portions represented by the following structures: 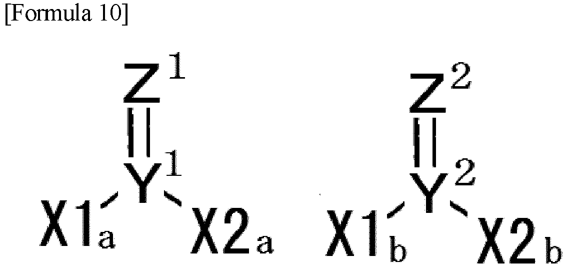 are preferably any one of the following portions, but are not limited thereto:

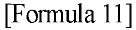

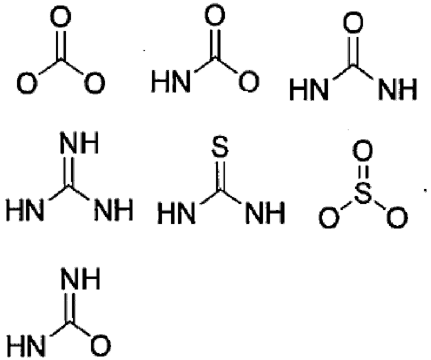

X1a, X1b, X2a and X2b preferably represent NH; Y¹ and Y² preferably represent C; Z¹ and Z² preferably represent NH; and V¹ and V² preferably represent S.

L₁ and L₂ each independently represent a divalent linking group consisting of a combination of groups selected from -CONH-, -NHCO-, -COO-, -OCO-, -CO-, -O-, and an alkylene group containing 1 to 10 carbon atoms.

Preferably, L₁ and L₂ each independently represent a divalent linking group consisting of a combination of groups selected from -CONH-, -NHCO-, -O-, and an alkylene group containing 1 to 10 carbon atoms.

Preferably, L₁ and L₂ each independently represent a divalent linking group consisting of a combination of groups selected from -CONH-, -NHCO-, and an alkylene group containing 1 to 10 carbon atoms.

L₄ represents a trivalent linking group, and is preferably the following: 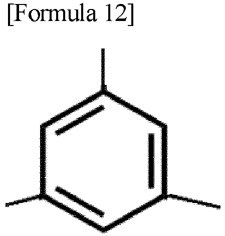 or, 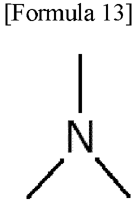 (which is a benzene-derived trivalent linking group or a nitrogen atom).

L₃ is preferably a group consisting of a combination of groups selected from -CONH-, -NHCO-, -COO-, -OCO-, -CO-, -O-, and an alkylene group containing 1 to 10 carbon atoms, and further comprising an amino group at the terminus.

### (2) Phthalocyanine dye

The phthalocyanine dye is preferably a silicon phthalocyanine dye. Specific examples of the phthalocyanine dye, such as IRDye (registered trademark) 700DX, are described in, for example, U.S. Patent No. 7,005,518.

As such a phthalocyanine dye, a dye represented by the following formula (21) can be used. As an example, a dye represented by the following formula (22) can be used.

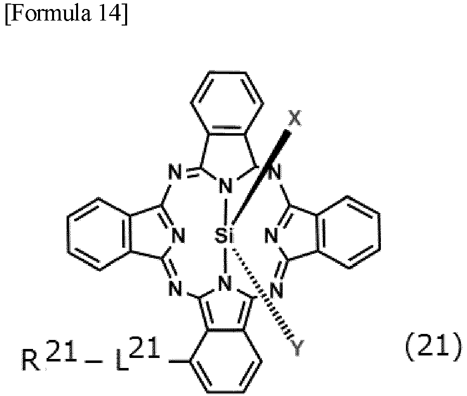

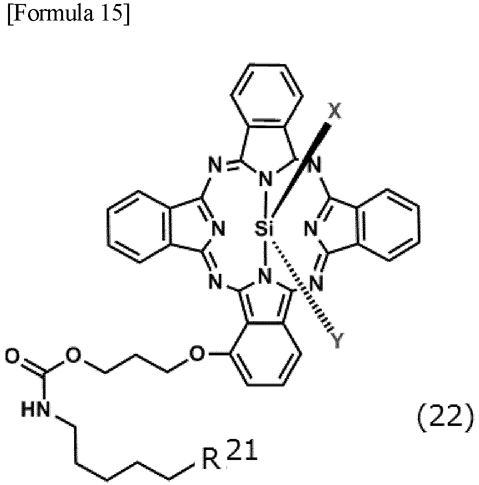

In the above formulae, L²¹ represents a divalent linking group, and R²¹ represents a functional group capable of binding to the compound represented by the formula (1) or a salt thereof.

In the above formulae, X and Y each independently represent a hydrophilic group, -OH, a hydrogen atom, or a substituent. Examples of the substituent used herein may include, but are not particularly limited to, a halogen atom (a fluorine atom), a substituent containing a carbon atom (a hydrocarbo group, etc.), and a substituent containing a nitrogen atom (an amino group, etc.).

Specific examples of X and Y may include:
(i) a case where both X and Y are hydrophilic groups;
(ii) a case where either X or Y is a hydrophilic group, and the other is -OH or a hydrogen atom; and
(iii) a case where X and Y are -OH or hydrogen atoms.

The hydrophilic group(s) represented by X and/or Y are not particularly limited. One example is shown below.

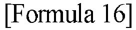

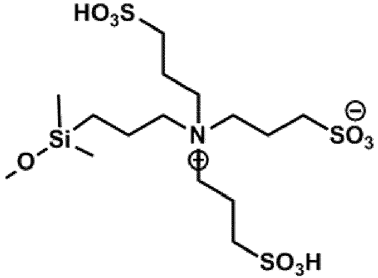

As a phthalocyanine dye, a commercially available product such as IRDye (registered trademark) 700DX can be used. In the present invention, an NHS ester of IRDye (registered trademark) 700DX is used, and is allowed to react with a biotin-modified dimer having an amino group to produce a conjugate. Other variations of IRDye (registered trademark) 700DX are described in U.S. Patent No. 7,005,518, and those can also be used.

R²¹ represents a functional group capable of binding to the compound represented by the formula (1) or a salt thereof. R²¹ is preferably a functional group that can react with a carboxyl group, amine or a thiol group on the biotin-modified dimer and can bind thereto. Preferred examples of R²¹ may include, but are not particularly limited to, activated ester, halogenated acyl, halogenated alkyl, appropriately substituted amine, anhydride, carboxylic acid, carbodiimide, hydroxyl, iodoacetamide, isocyanate, isothiocyanate, maleimide, NHS ester, phosphoramidite, sulfonic acid ester, thiol, and thiocyanate.

L²¹ represents a divalent linking group, and for example, it may include: any given combination of an ether, thioether, amine, ester, carbamate, urea, thiourea, oxy or amide bond, or a single, double, triple or aromatic carbon-carbon bond; or a phosphorus-oxygen, phosphorus-sulfur, nitrogen-nitrogen, nitrogen-oxygen, or nitrogen-platinum bond; or an aromatic or heteroaromatic bond. L²¹ is preferably a group consisting of a combination of groups selected from -CONH-, -NHCO-, -COO-, -OCO-, -CO-, -O-, and an alkylene group containing 1 to 10 carbon atoms.

-L²¹-R²¹ may include a phosphoramidite group, NHS ester, active carboxylic acid, thiocyanate, isothiocyanate, maleimide, and iodoacetamide.

L²¹ contains a -(CH₂)ₙ- group, and in the formula, n is an integer of 1 to 10, and is preferably an integer of 1 to 4. As one example, -L²¹-R²¹ is -O-(CH₂)₃-OC(O)-NH-(CH₂)₅-C(O)O-N-succinimidyl.

### (3) Therapeutic kit using conjugate of biotin-modified dimer and phthalocyanine dye

According to the present invention, provided is a therapeutic kit, in which the conjugate of a biotin-modified dimer and a phthalocyanine dye of the present invention is combined with a mutant streptavidin-molecular probe conjugate.

As mutant streptavidins used herein, those described in International Publication WO2014/129446 and International Publication WO2015/125820 can be used. Particularly preferably, the mutant streptavidin LISA314-V2122 described in Example 3 of International Publication WO2015/125820 (SEQ ID NO: 4 of International Publication WO2015/125820) (SEQ ID NO: 1 of the description of the present application) can be used.

Examples of the molecular probe used herein may include an antibody, a peptide, a nucleic acid, and an aptamer. Specifically, an antibody, a peptide, a nucleic acid, an aptamer, etc., which target the following antigens specifically expressed in cancer, can be used:

Epiregulin (EREG), ROBO 1, 2, 3, and 4, 1-40-β-amyloid, 4-1BB, 5AC, 5T4, ACVR2B, adenocarcinoma antigen, α-fetoprotein, angiopoetin 2, anthrax toxin, AOC3 (VAP-1), B-lymphoma cells, B7-H3, BAFF, β amyloid, C242 antigen, C5, CA-125, carbonic anhydrase 9 (CA-IX), cardiac myosin, CCL11 (eotaxin-1), CCR4, CCR5, CD11, CD18, CD125, CD140a, CD147 (basigin), CD147 (basigin), CD15, CD152, CD154 (CD40L), CD154, CD19, CD2, CD20, CD200, CD22, CD221, CD23 (IgE receptor), CD25 (IL-2 receptor α chain), CD28, CD3, CD30 (TNFRSF8), CD33, CD37, CD38 (cyclic ADP ribose hydrolase), CD4, CD40, CD41 (integrin α-IIb), CD44 v6, CD5, CD51, CD52, CD56, CD6, CD70, CD74, CD79B, CD80, CEA, CFD, ch4D5, CLDN18.2, *Clostridium difficile*, clumping factor A, CSF2, CTLA-4, cytomegalovirus, cytomegalovirus glycoprotein B, DLL4, DR5, *E. coli* Shiga toxin type 1, *E. coli* Shiga toxin type 2, EGFL7, EGFR, endotoxin, EpCAM, episialin, ERBB3, *Escherichia coli,* F protein of respiratory syncytial virus, FAP, fibrin II β chain, fibronectin extra domain-B, folate receptor 1, Frizzled receptor, GD2, GD3 ganglioside, GMCSF receptor α chain, GPNMB, hepatitis B surface antigen, hepatitis B virus, HER1, HER2/neu, HER3, HGF, HIV-1, HLA-DRβ, HNGF, Hsp90, human β amyloid, human scatter factor receptor kinase, human TNF, ICAM-1 (CD54), IFN-α, IFN-γ, IgE, IgE Fc region, IGF-1 receptor, IGF-I, IgG4, IGHE, IL-1β, IL-12, IL-13, IL-17, IL-17A, IL-22, IL-23, IL-4, IL-5, IL-6, IL-6 receptor, IL-9, ILGF2, influenza A hemagglutinin, insulin-like growth factor I receptor, integrin α4, integrin α4β7, integrin α5β1, integrin α7β7, integrin αIIbβ3, integrin αvβ3, integrin γ induced protein, interferon receptor, interferon α/β receptor, ITGA2, ITGB2 (CD18), KIR2D, L-selectin (CD62L), Lewis-Y antigen, LFA-1 (CD11a), lipoteichoic acid, LOXL2, LTA, MCP-1, mesothelin, MS4A1, MUC1, mucin CanAg, myostatin, N-glycolylneuraminic acid, NARP-1, NCA-90 (granulocyte antigen), NGF, NOGO-A, NRP1, *Oryctolagus cuniculus,* OX-40, oxLDL, PCSK9, PD-1, PDCD1, PDGF-R α, phosphatidylserine, prostate cancer cells, *Pseudomonas aeruginosa*, Rabies virus glycoprotein, RANKL, respiratory syncytial virus, RHD, Rh (Rhesus) factor, RON, RTN4, sclerostin, SDC1, selectin P, SLAMF7, SOST, sphingosine-1-phosphate, TAG-72, TEM1, tenascin C, TFPI, TGFβ1, TGFβ2, TGF-β, TNF-α, TRAIL-R1, TRAIL-R2, tumor antigen CTAA16.88, MUC1 tumor-specific glycosylation, TWEAK receptor, TYRP1 (glycoprotein 75), VEGF-A, VEGFR-1, VEGFR2, vimentin, and VWF

Among the above-described antigens, epiregulin (EREG), CEA, and HER2 are preferable.

A fusion body of a molecular probe such as a tumor antigen-specific antibody molecule and a mutant streptavidin is prepared, and the prepared fusion body is then administered to a patient, so that the mutant streptavidin can be accumulated specifically in cancer cells. Subsequently, a conjugate of a biotin-modified dimer having an affinity for the above-described mutant streptavidin and a phthalocyanine dye is administered to the patient, so that the phthalocyanine dye can be accumulated exactly in the cancer cells.

Otherwise, in the present invention, a complex is prepared by binding a conjugate of a molecular probe such as a tumor antigen-specific antibody molecule and a mutant streptavidin with a conjugate of a biotin-modified dimer and a phthalocyanine dye, and the thus prepared complex can be administered to a patient.

Various types of molecules can be used as antibodies that are to be bound to the mutant streptavidin. Either a polyclonal antibody or a monoclonal antibody may be used. The subclass of the antibody is not particularly limited. Preferably, IgG, and particularly preferably, IgG₁ is used. Furthermore, the term "antibody" includes all of modified antibodies and antibody fragments. Examples of such an antibody include, but are not limited to: a humanized antibody; a human type antibody; a human antibody; antibodies from various types of animals such as a mouse, a rabbit, a rat, a guinea pig and a monkey; a chimeric antibody between a human antibody and an antibody from a different type of animal; diabody; scFv; Fd; Fab; Fab'; and F(ab)'₂.

The conjugate of the mutant streptavidin and the antibody can be obtained by applying a method known to persons skilled in the art. For example, such a conjugate can be obtained by a chemical bond method (US5,608,060). Alternatively, DNA encoding the mutant streptavidin is ligated to DNA encoding an antibody, and using an expression vector or the like, the ligated DNA is then expressed in a host cell, so that such a conjugate can be obtained in the form of a fusion protein. The DNA encoding the mutant streptavidin may be ligated to the DNA encoding an antibody via DNA encoding a suitable peptide, called a linker. The mutant streptavidin-molecular probe conjugate is desirably produced, while keeping the specific binding force between an antibody and a target molecule.

### (4) Photoimmunotherapy

The conjugate of a biotin-modified dimer and a phthalocyanine dye according to the present invention is administered to a subject, and the cells are then irradiated with an excitation light in an amount effective for suppression of cell proliferation or induction of cell death, so that the cell proliferation can be suppressed or the cell death can be induced, and thereby the subject can be treated.

Preferably a complex of the conjugate of a biotin-modified dimer and a phthalocyanine dye according to the present invention and a mutant streptavidin-molecular probe conjugate is administered to a subject, and the cells are then irradiated with an excitation light in an amount effective for suppression of cell proliferation or induction of cell death, so that the cell proliferation can be suppressed or the cell death can be induced, and thereby the subject can be treated.

The subject includes humans and non-human animals. Examples of the subject may include humans and experimental animals such as mice. The subject is preferably affected with a disease regarding which suppression of cell proliferation or induction of cell death is desired. For example, the subject is affected with cancer or solid tumor.

Examples of the "cancer" may include carcinoma, lymphoma, blastoma, sarcoma, and leukemia or malignant lymphoma. Specific examples of the cancer may include squamous cell carcinoma (e.g., epithelial squamous cell carcinoma), lung cancer including small cell lung cancer, non-small cell lung cancer ("NSCLC"), pulmonary adenocarcinoma and pulmonary squamous cell carcinoma, peritoneal cancer, hepatocarcinoma, corpus ventriculi or stomach cancer, including digestive cancer, pancreatic cancer, glioblastoma, cervical cancer, ovarian cancer, liver cancer, bladder cancer, hepatocellular cancer, breast cancer, colon cancer, rectal cancer, colorectal cancer, endometrial membrane cancer or endometrial carcinoma, salivary gland carcinoma, kidney or renal region cancer, prostate cancer, vulvar cancer, thyroid cancer, hepatocellular carcinoma, anal carcinoma, penile carcinoma, and head and neck cancer.

The solid tumor means a benign or malignant, abnormal cell mass that generally does not contain a capsule. Examples of the solid tumor may include glioma, astrocytoma, medulloblastoma, craniopharyngioma, ependymoma, pineal gland tumor, hemangioblastoma, acoustic neuroma, oligodendrocyte, meningioma, melanoma, neuroblastoma, and retinoblastoma.

In the photoimmunotherapy, the "conjugate of a biotin-modified dimer and a phthalocyanine dye" or the "complex of the conjugate of a biotin-modified dimer and a phthalocyanine dye and a mutant streptavidin-molecular probe conjugate" is administered to a subject, and thereafter, the subject is irradiated with a light, so that the subject can be treated.

Examples of the administration method to the subject may include, but are not limited to, a local route, an injection (a subcutaneous injection, an intramuscular injection, an intradermal injection, an intraperitoneal injection, an intratumoral injection, an intravenous injection, etc.), an oral route, an ocular route, a sublingual route, a rectal route, a percutaneous route, an intranasal route, a vaginal route, and an inhalation route.

The "conjugate of a biotin-modified dimer and a phthalocyanine dye" or the "complex of the conjugate of a biotin-modified dimer and a phthalocyanine dye and a mutant streptavidin-molecular probe conjugate" is preferably administered in a therapeutically effective amount. The therapeutically effective amount per 60 kg is at least 0.5 mg (mg/60 kg), at least 5 mg/60 kg, at least 10 mg/60 kg, at least 20 mg/60 kg, at least 30 mg/60 kg, or at least 50 mg/60 kg. For example, when it is intravenously administered, the applied dose is 1 mg/60 kg, 2 mg/60 kg, 5 mg/60 kg, 20 mg/60 kg, or 50 mg/60 kg, and it is, for example, 0.5 to 50 mg/60 kg. In another example, the therapeutically effective amount is at least 100 µg/kg, at least 500 µg/kg or at least 500 µg/kg, and it is, for example, at least 10 µg/kg. For example, when it is intratumorally or intraperitoneally administered, the dose is 100 µg/kg, 250 µg/kg, approximately 500 µg/kg, 750 µg/kg, or 1000 µg/kg, and it is, for example, 10 µg/kg to 1000 µg/kg, In one example, when it is administered in the form of a solution for local administration, the therapeutically effective amount is 10 µg/ml, 20 µg/ml, 30 µg/ml, 40 µg/ml, 50 µg/ml, 60 µg/ml, 70 µg/ml, 80 µg/ml, 90 µg/ml, 100 µg/ml or the like, or it is 20 µg/ml to 100 µg/ml, or it is at least 500 µg/ml, or at least 1 µg/ml.

The above-described dose can be administered once or divided doses over several administrations (2, 3, or 4 times, etc.), or as a single preparation.

The "conjugate of a biotin-modified dimer and a phthalocyanine dye" or the "complex of the conjugate of a biotin-modified dimer and a phthalocyanine dye and a mutant streptavidin-molecular probe conjugate" can be administered alone, or can also be administered in the presence of a pharmaceutically acceptable carrier, or can also be administered in the presence of other therapeutic agents (other anticancer agents, etc.).

The "conjugate of a biotin-modified dimer and a phthalocyanine dye" or the "complex of the conjugate of a biotin-modified dimer and a phthalocyanine dye and a mutant streptavidin-molecular probe conjugate" can bind to target cells or target tissues, such as circulating tumor cells or solid tumor cells. Thereafter, the target cells or tissues are irradiated with a light, so that the above-described conjugate or complex can absorb the light and can damage or destroy the target cells or tissues.

In the photoimmunotherapy, the wavelength of the irradiation light is preferably 660 to 740 nm, and the irradiation light has a wavelength of, for example, 660 nm, 670 nm, 680 nm, 690 nm, 700 nm, 710 nm, 720 nm, 730 nm, or 740 nm. Light irradiation may be carried out using a device equipped with a near infrared (NIR) light emitting diode.

The light irradiation amount is at least 1 J/cm², for example, at least 4 J/cm², at least 10 J/cm², at least 15 J/cm², at least 20 J/cm², at least 50 J/cm², or at least 100 J/cm². It is, for example, 1 to 500 J/cm². Light irradiation may be carried out several times (e.g., 2, 3, 4, 5, 6, 7, 8, 9, or 10 times).

The present invention will be more specifically described in the following examples. However, these examples are not intended to limit the scope of the present invention.

### Examples

### < Production Example 1 >

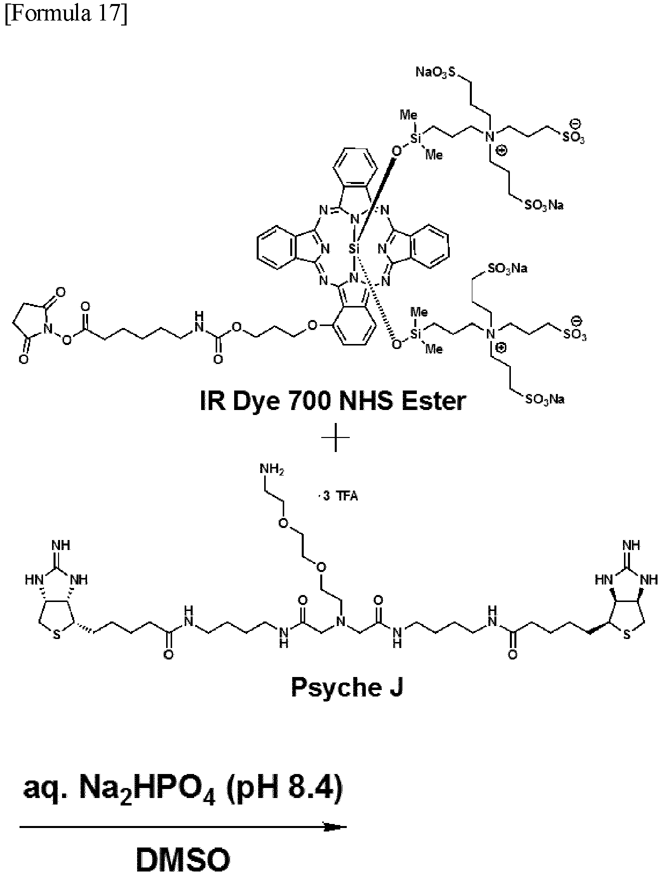

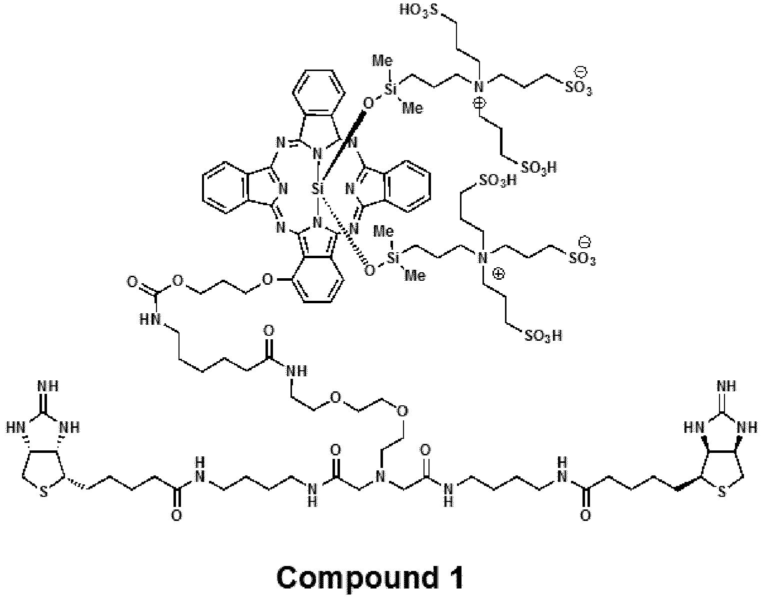

IR Dye 700 NHS Ester (3.0 mg, 1.5 µmol), disodium hydrogen phosphate buffer (pH 8.4, 144 µL) and dimethyl sulfoxide (120 µL) were added to Psyche J (1.8 mg, 1.6 µmol), light was then shielded with an aluminum foil, and the mixture was then stirred at room temperature for 24 hours. Thereafter, the reaction solution diluted with water to 500 µL was purified by reverse phase HPLC (gradient: 0% for 5 min; 0-100% for 100 min acetonitrile in a 50 mM triethylammonium acetate aqueous solution (pH 6.5), retention time = 47.2 min, YMC-Triart C18, flow rate = 3.5 mL/min) to obtain a target compound **1** (dark blue green). Quantification was carried out from the reported molar absorption coefficient of IR700 at 694 nm in water (165,000 (M⁻¹ cm⁻¹)), and the yield (1.0 µmol, 72%) was calculated.
LRMS (ESI): *m*/*z* 1303 [M+2H]²⁺, 869 [M+3H]³⁺

### < Production Example 2 >

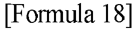

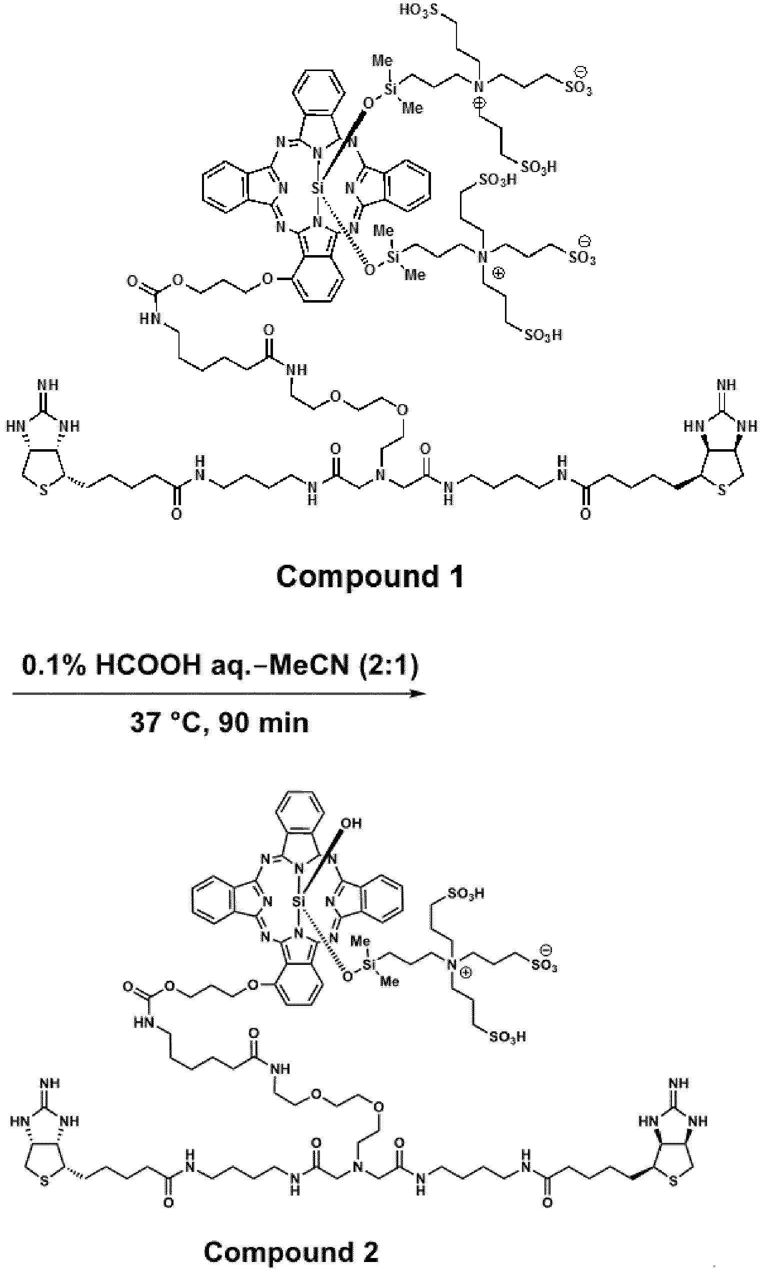

A 0.1% formic acid aqueous solution (266 µL) and acetonitrile (133 µL) were added to the compound **1** (0.375 mg, 144 nmol), and the obtained mixture was stirred with a Vortex mixer and was then centrifuged. The resultant was left at rest under conditions of in a darkroom at 37°C for 90 minutes. The resulting solution was purified by reverse phase HPLC (gradient: 0% for 5 min; 0-100% for 100 min acetonitrile in a 50 mM triethylammonium acetate aqueous solution (pH 6.5), retention time = 51.6 min, YMC-Triart C18, flow rate = 3.5 mL/min) to obtain a target compound **2** (dark blue green). Quantification was carried out in the same manner as described above, and the yield (48 nmol, 33%) was calculated.
LRMS (ESI): *m*/*z* 1054 [M-H₂O+2H]²⁺, 703 [M-H₂O+3H]³⁺

### < Production Example 3 >

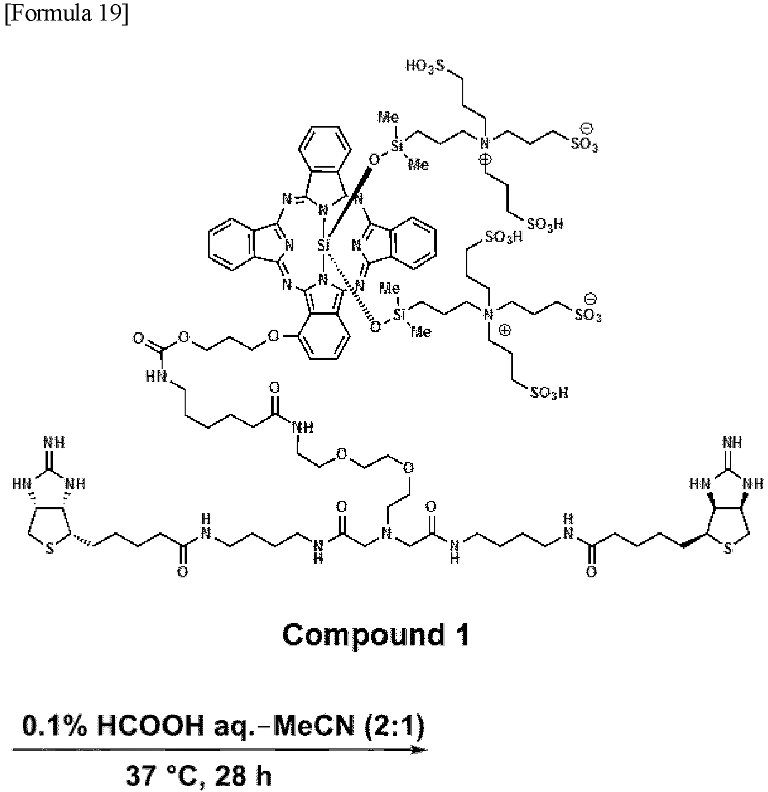

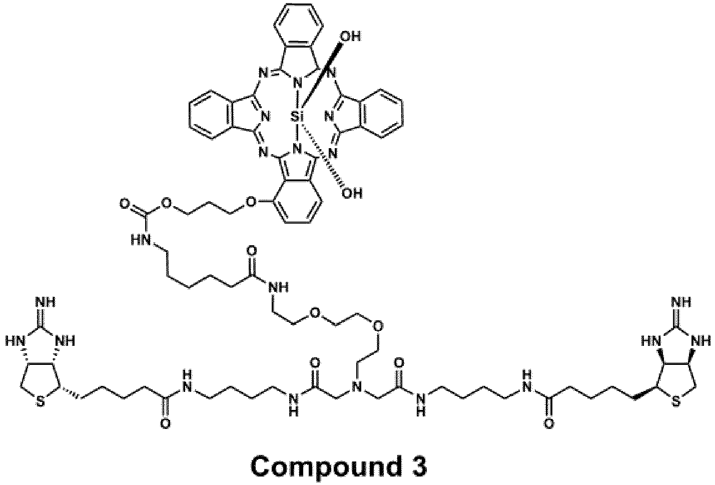

A 0.1% formic acid aqueous solution (266 µL) and acetonitrile (133 µL) were added to the compound **1** (0.375 mg, 144 nmol), and the obtained mixture was stirred with a Vortex mixer and was then centrifuged. The resultant was left at rest under conditions of in a darkroom at 37°C for 28 hours. The resulting solution was purified by reverse phase HPLC (gradient: 0% for 5 min; 0-100% for 100 min acetonitrile in a 50 mM triethylammonium acetate aqueous solution (pH 6.5), retention time = 56.2 min, YMC-Triart C18, flow rate = 3.5 mL/min) to obtain a target compound **3** (dark blue green). Quantification was carried out in the same manner as described above, and the yield (15 nmol, 10%) was calculated.
LRMS (ESI): *m*/*z* 813 [M-H₂O+2H]²⁺, 542 [M-H₂O+3H]³⁺

### Test Example 1:

### < Preparation of scFv-Cupid molecule expression vector >

The sequences of a gene ST01 (the nucleotide sequence and amino acid sequence thereof are as set forth in SEQ ID NO: 2 and SEQ ID NO: 3, respectively), which fuses an scFv-type antibody recognizing an EREG antigen with a Cupid molecule (the amino acid sequence thereof is as set forth in SEQ ID NO: 1 in the sequence listing) and expresses a fusion protein in *Escherichia coli*, and the gene sequences of a chaperone molecule skp, were obtained by artificial synthesis. For the expression of the protein in *Escherichia coli,* a pETDuet-1 vector was used. Specifically, the artificially synthesized chaperone skp gene sequences (the nucleotide sequence and amino acid sequence thereof are as set forth in SEQ ID NO: 4 and SEQ ID NO: 5, respectively) were amplified by PCR using the primers (skp_Fw: TACATATGGATAAAATTGCCATTGTTAAT (SEQ ID NO: 6), and skp_Rv: TTTTATCCATATGTATATCTCCTTC (SEQ ID NO: 7)), and a band was separated by electrophoresis and was then purified. As such a pETDuet-1 vector, a vector linearized at the MCS2 Ndel site by a treatment with the restriction enzyme Ndel was produced. In accordance with the protocol and amount of In-Fusion HD Cloning Kit (Takara Bio, Inc.), skp was ligated to the linearized vector, and it was then confirmed by cloning and sequence analysis that a sequence of interest had been incorporated. The sequence of interest was incorporated into the MCS2 of the same vector to obtain an expression vector pETDuet_skp.

Thereafter, ST01 was incorporated into pETDuet_skp as follows. Using pETDuet_skp as a template, the vector was linearized using the primers (Linear1 Rv: GGTATATCTCCTTCTTAAAG (SEQ ID NO: 8), and Linear1 Fw: AATTCGAGCTCGGCGCGCCTG (SEQ ID NO: 9)). At the same time, the artificially synthesized ST01 gene was amplified by PCR using the primers (ST01 Fw: AGAAGGAGATATACCATGAAATATCTGCTGCCGAC (SEQ ID NO: 10), and ST01 Rv: CGCCGAGCTCGAATTTTAATGATGGTGATGATGATG (SEQ ID NO: 11)). The gel was cut out and was then purified to prepare an insert. The linearized vector and the insert were cloned in accordance with the protocol and amount of In-Fusion HD Cloning Kit (Takara Bio, Inc.), and thereafter, it was confirmed by sequence analysis that the incorporated gene was a correct one, which was then used as an ST01 expression vector.

### < Expression and purification of scFv-Cupid protein >

A plasmid vector, which was confirmed by cloning and sequence analysis that a gene of interest had been incorporated therein, was introduced into competent cells BL21 (DE3) (ECOS Competent *E. coli* BL21 (DE3), Nippon Gene Co., Ltd.), so that the cells were transformed. A culture medium prepared by culturing the cells in 100 mL of 2 x YT medium overnight was inoculated into 1 L of a culture solution, and the obtained mixture was then cultured at 37°C. When the OD value at 600 nm exceeded 2.0, IPTG was added to the culture to a final concentration of 0.5 mM, so that the expression of the protein was induced.

Regarding ST01, after induction of the expression with IPTG, it was cultured at 16°C overnight, and thereafter, a culture supernatant was recovered.

Using Ni-NTA resin (cOmplete His-Tag Purification Resin, SIGMA-ALDRICH), the recovered culture supernatant was subjected to affinity purification with 6xHis-Tag added to the C-terminus of the protein according to a batch method, so as to obtain a roughly purified product. Furthermore, using Protein L resin (Capto L, GE Healthcare), affinity purification with a κ light chain was carried out. In this operation, as a binding/washing buffer, PBS was used, and as an elution buffer, a glycine-hydrochloric acid solution (10 mM, pH 2.0) was used. The purified product was concentrated with a centrifugal filtration filter (Vivaspin Turbo 15 100K MWCO, Sartorius). After completion of the concentration, a disposable column for buffer exchange (PD-10, GE Healthcare) was used to replace the buffer for the product with PBS.

### < Preparation of cultured cells >

DLD-1 cells (EREG-positive) (human colon adenocarcinoma-derived cells) were cultured in 10% FBS RPMI1640 (FUJIFILM Wako Pure Chemical Corporation). For a cytotoxicity test, the cells were seeded on a 96-well plate at a cell density of 3 x 10³ to 5 x 10³ cells per well. These cells were prepared the day before the cytotoxicity test.

### < Preparation of pre-conjugate >

Each ST01 and compounds 1, 2 and 3 dissolved in 100% DMSO were each mixed with each other at a molar ratio of 1 : 2 before dilution of the medium, and the obtained mixtures were then incubated at room temperature for 10 minutes. Thereafter, the reaction mixtures were diluted with a culture solution (10% FBS RPMI1640), so that the final concentration of scFv-Cupid could be 10 µg/mL (60 to 61 nM) and the final concentration of the Psyche compound could be 120 nM. Thereafter, the thus obtained mixtures were incubated at room temperature for 10 minutes to obtain test solutions. It is to be noted that a culture solution was used as a control without addition of scFv-Cupid, whereas DMSO was used as a control of compound.

### < Test using cells >

The culture solution for the cells cultured the day before the cytotoxicity test, as mentioned above, was discarded, and 100 µL of the test solution was then added to each well, followed by performing culture for 4 hours. Thereafter, the resultant was irradiated with a light having a peak wavelength of 690 nm ± 10 nm. A measurement apparatus, in which a sensor with 400 to 1100 nm was connected with a photo power meter PM121D (both of which are manufactured by THORLABS), was used to measure an irradiation energy on the culture plate, and the irradiation time was then determined. The irradiation energy and the irradiation time were set to be 1.1 J/cm² (2 min), 4.5 J/cm² (8 min), and 18.4 J/cm² (30 min). After completion of the irradiation at each irradiation time, culture was carried out in 5% CO₂ at 37°C, and 48 hours later, cell proliferation was assayed using Cell Counting Kit-8 (DOJINDO LABORATORIES). Specifically, 10 µL of Cell Counting Kit-8 solution was added into each well, and it was then incubated in 5% CO₂ at 37°C for 1 to 2 hours. Thereafter, the absorbance at 450 nm was measured using an absorption spectrometer. A well containing only the medium was defined to be a background (BG), and a well containing only DMSO was defined to be 100% control (Cont.). The value of cell proliferation % under individual culture conditions was calculated according to the following expression: 100 x (measurement value A - BG) / (Cont. - BG) (unit: %).

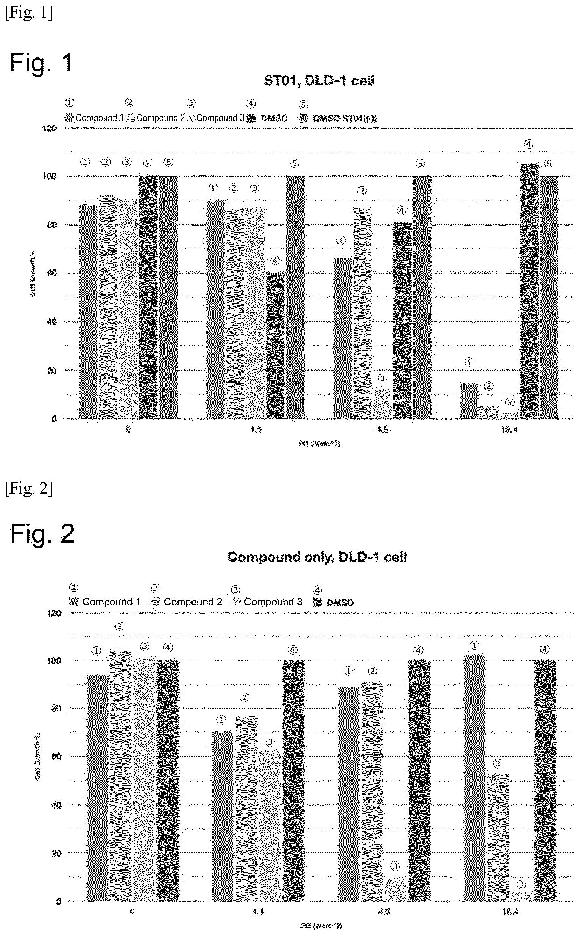

In Fig. 1, suppression of the cell proliferation, which was dependent on pre-incubation with ST01 and was also dependent on light irradiation, was confirmed.

In Fig. 2, it was confirmed that even in a case where pre-incubation with ST01 is not performed, suppression of the cell proliferation may be confirmed in some cases.
< SEQ ID NO: 2: nucleotide sequence of ST01 > 
ATGAAATACCTATTGCCTACGGCAGCCGCTGGATTGTTATTACTCGCGGCCCAGCCGGC
CATGGCCCAGGTGCAGCTGCAGCAGTCTGGCGCCGAAGTGAAGAAACCTGGCGCCTC
 
CGTGAAGGTGTCCTGCAAGGCCTCCGGCTTCAACATCAAGGACACCTACATGCACTGG
GTGCGACAGGCCCCTGAGCAGGGCCTGGAATGGATGGGCAGAATCGACCCCCTGAAC
GACAAGACTAAGTACGACCCCAAGTTCCAGGGCAGAGTGACCATCACCGCCGACACC
TCTACCAACACCGCCTACCTGGAACTGTCCTCCCTGACCTCTGAGGACACCGCCGTGT
ACTACTGCGCTAGAGGCGGCGGAGATCCCGTGTTCGTGTATTGGGGCCAGGGCACCCT
CGTGACCGTGTCTGCTTCTTCTGGCGGAGGCGGATCTGGGGGCGGAGGTTCTGGTGGT
GGTGGAAGCGGTGGCGGTGGATCTGGCGGCGATATCCAGATGACCCAGTCCCCCAGCT
CCCTGTCTGCCTCTGTGGGCGACCGCGTGACCATTACATGCAAGGCCAGCCAGGACAT
CAACAAGTACCTGGCCTGGTATCAGCACAAGCCCGGCCAGGCTCCTCGGCTGCTGATC
CACTATACCTCCACCCTGCACCCCGGCATCCCTTCCAGATTCTCCGGCTCTGGCTCCGG
CACCGACTTTACCTTCTCCATCTCCAGCCTGCAGCCCGAGGATATCGCTACCTACTACT
GCCTGCAGTACGACAACCTGCGGACCTTCGGAGGCGGCACCAAGGTGGAAATCAAGC
GGACCAGCGGCGGAGGTGGAAGCGGTGGAGGTGGAGCCGAAGCAGGTATTACCGGC
ACCTGGAGCGATCAGCTGGGCGATACCTTTATTGTGACCGCCGGCGCAGATGGTGCGC
TGACCGGCACCTATGAAAATGCCGTGGGTGGTGCGGAAAGCCGTTATGTTCTGACCGG
TCGTTATGATAGCGCACCGGCAACCGATGGCAGCGGCACCGCCCTGGGTTGGACCGTG
GCGTGGAAAAACAATAGCAAAAACGCCCATAGCGCGACCACCTGGAGCGGCCAGTAT
GTTGGCGGTGCCGATGCGAAAATTAACACCCAGTGGCTGCTGACCAGCGGCACCACC
AATGCCAATGCGTGGAAAAGCACCCTGGTGGGTCATGATACCTTTACCAAAGTTAAAC
CGAGCGCGGCCAGCCACCACCACCACCACCACTGA
< SEQ ID NO: 4: nucleotide sequence of skp >

GATAAAATTGCCATTGTTAATATGGGTAGCCTGTTTCAGCAGGTTGCACAGAAAACCGG
TGTTAGCAATACCCTGGAAAATGAATTTAAAGGTCGTGCAAGCGAACTGCAGCGTATG
GAAACCGATCTGCAGGCAAAAATGAAAAAACTGCAGAGCATGAAAGCAGGTAGCGAT
CGTACCAAACTGGAAAAAGATGTTATGGCACAGCGTCAGACCTTTGCCCAGAAAGCA
CAGGCATTTGAACAGGATCGTGCACGTCGTAGCAATGAAGAACGTGGTAAACTGGTTA
CCCGTATTCAGACCGCAGTTAAAAGCGTTGCAAATAGCCAGGATATTGATCTGGTTGTT
GATGCAAATGCCGTTGCCTATAATAGCAGTGATGTGAAAGATATTACCGCAGACGTTCT

GAAACAAGTGAAATAA

### Example 2: Expression and purification of CEA-V2122 protein

V2122 is a mutant streptavidin described in Example 3 of International Publication WO2015/125820 (SEQ ID NO: 4 shown in International Publication WO2015/125820). The amino acid sequence of V2122 (a sequence having a 6xHis tag at the C-terminus) is as set forth in SEQ ID NO: 12 in the sequence listing.

scFv-V2122 is prepared by binding a single-chain antibody (scFv) against CEACAM5 with the above-described V2122. This scFv-type anti-CEACAM5 antibody is an scFv sequence described in a patent document US7626011B2. The amino acid sequence of the scFv-type anti-CEACAM5 antibody is as set forth in SEQ ID NO: 13 in the sequence listing. In addition, the amino acid sequence of CEA-V2122 prepared by binding the scFv-type anti-CEACAM5 antibody with V2122 via an amino acid linker (GGGGSGGGG) (SEQ ID NO: 22) is as set forth in SEQ ID NO: 14 in the sequence listing.

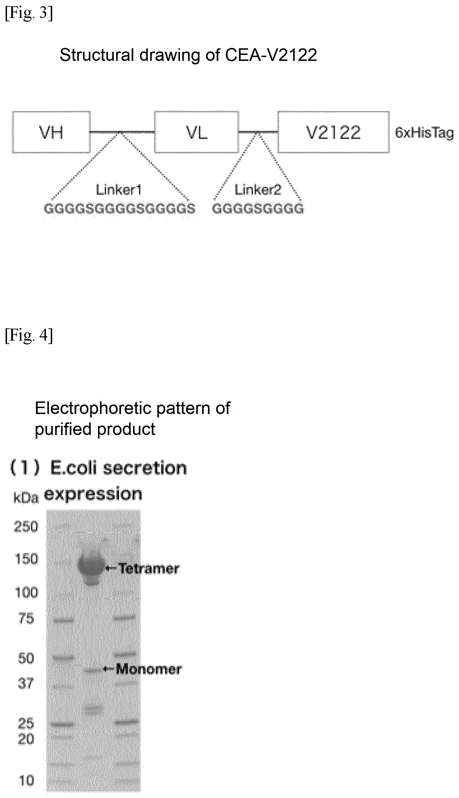

For the expression of a CEA-V2122 fusion protein, the DNA codon of a CEA-V2122 gene sequence, in which a pelB signal for secretion and expression in *Escherichia coli* had been incorporated into the N-terminus and a 6xHis-Tag sequence had been incorporated into the C-terminus, was optimized for *Escherichia coli,* thereby synthesizing an artificial gene. This amino acid sequence is as set forth in SEQ ID NO: 15 in the sequence listing, and the DNA sequence is as set forth in SEQ ID NO: 16 in the sequence listing. Moreover, an outline of a domain structure is shown in Fig. 3.

As a specific protein expression vector, a vector prepared by incorporating a chaperone skp gene into MCS2 of a pETDuet1 vector was used. Regarding the skp gene, the DNA codon was optimized for *Escherichia coli* based on the amino acid sequence as set forth in SEQ ID NO: 17 in the sequence listing, thereby synthesizing an artificial gene. The synthesized skp gene was amplified by PCR, using the primers (AAGGAGATATACATATGGATAAAATTGCCATTGTTAATAT (SEQ ID NO: 23), and TTGAGATCTGCCATATGTTATTTCACTTGTTTCAGAACG (SEQ ID NO: 24)), and the amplified gene was then cloned into MCS2 of the pETDue1 vector linearized with the restriction enzyme NdeI, using In-Fusion HD Cloning Kit, so as to obtain pETDuet_skp. Subsequently, the CEA-V2122 gene was incorporated into MCS1 of pETDuet_skp. Specifically, the artificially synthesized CEA-V2122 gene was amplified by PCR, using the primers (AGAAGGAGATATACCATGAAATATCTGCTGCCGAC (SEQ ID NO: 25), and CGCCGAGCTCGAATTTTAATGATGGTGATGATGATG (SEQ ID NO: 26)). Moreover, pETDuet_skp was linearized by PCR, using the primers (GGTATATCTCCTTCTTAAAGTTAAAC (SEQ ID NO: 27), and AATTCGAGCTCGGCGCGCCTGCAG (SEQ ID NO: 28)). The CEA-V2122 amplified by PCR and the linearized pETDuet_skp were subjected to cloning using In-Fusion HD Cloning Kit. The cloned vector was confirmed by sequencing, in terms of the gene sequence incorporated therein, and thereafter, it was referred to as pETDuet_CEA-V2122_skp.

For the expression of the protein, pETDuet_CEA-V2122_skp was transformed into BL21(DE3) (Nippon Gene Co., Ltd.), which was then pre-cultured in 2xYT medium (SIGMA-ADLRICH) at 37°C overnight. The medium used in the pre-culture was added to a new medium for 100-fold dilution, and culture was then carried out at 37°C, until OD (600 nm) became 0.5 to 2.0. Subsequently, IPTG was added to the culture to a final concentration of 0.5 mM, and the obtained mixture was then cultured at 37°C for 4 hours. Thereafter, a culture supernatant was recovered and was then preserved at 4°C.

The CEA-V2122 protein was roughly purified according to a batch method utilizing 6xHis-Tag added to the C-terminus. Specifically, cOmplete His-Tag Purification Resin equilibrated with buffer A (50 mM Tris-HCl, 0.2 M NaCl, 1 mM EDTA, and 5 mM Imidazole; pH 8.0) was added to the culture supernatant preserved at 4°C. The obtained mixture was stirred for 2 hours to overnight at 4° C, so that the protein was allowed to bind to the resin. Subsequently, the resin was recovered into a column, and a 20 column volume of washing operation was performed with buffer A. Thereafter, a roughly purified product of CEA-V2122 was recovered by elution with buffer B (50 mM Tris-HCl, 0.2 M NaCl, 1 mM EDTA, and 400 mM Imidazole; pH 8.0).

Subsequently, the roughly purified product was purified using a Protein L column. Specifically, 1 mL of Capto L (GE Healthcare Life Sciences) was filled into a PD-10 column, and was then equilibrated with 10 column volume of PBS, and the aforementioned roughly purified product was then applied thereto. Thereafter, the resultant was washed with 10 column volume of PBS, was then eluted with 10 mM glycine hydrochloride (pH 2.0), and was then subjected to centrifugal concentration using Vivaspin Turbo 15 (MWCO 100,000). Moreover, using PD-10 (GE Healthcare Life Science), the buffer was replaced with PBS, and centrifugal concentration was further carried out using Vivaspin Turbo 4 (MWCO 100,000) to obtain a finally purified product. After completion of SDS-PAGE electrophoresis, the purity of tetramer CEA-V2122 was assayed by CBB staining. The results are shown in Fig. 4. As an SDS-PAGE gel, Mini-PROTEAN TGX 4-15% (Bio-Rad) was used, and as a CBB staining solution, Bullet CBB Stain One (Ready To Use) (Nacalai Tesque, Inc.) was used.

From Fig. 4, it was confirmed that the purified CEA-V2122 comprises an approximately 150 kDa tetramer as a main component.

### Example 3: Evaluation of performance of CEA-V2122 by SPR

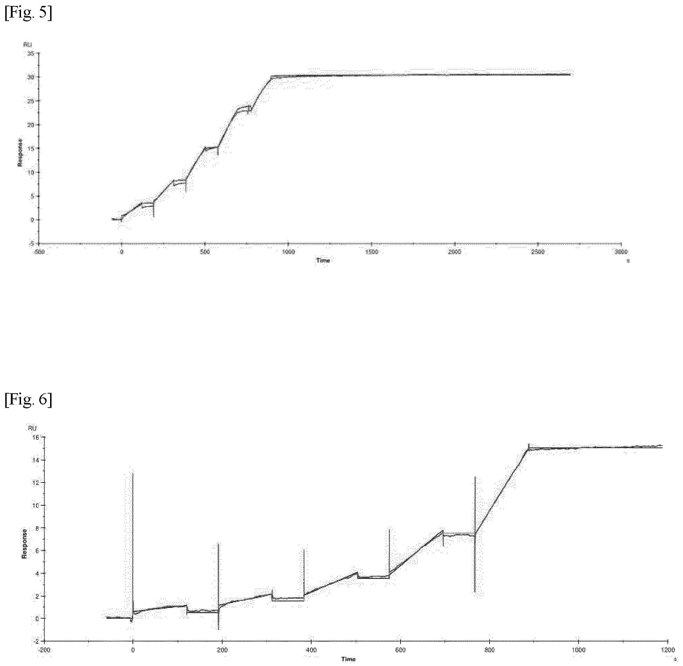

The affinity of CEA-V2122 for the antigen CEACAM5 was evaluated using a surface plasmon resonance (SPR) measuring device, Biacore T200 (GE Healthcare Life Sciences). Specifically, Recombinant Human CEACAM-5/CD66e Protein, CF (R & D SYSTEMS) was immobilized on Sensor Chip CM5 (GE Healthcare Life Sciences) using an amine-coupling kit (GE Healthcare Life Sciences). The final amount of the ligand immobilized was 279 RU. Moreover, with regard to the purified CEA-V2122, two-fold serial dilutions from 1E-08 M to 6.25E-10 M were prepared as analytes. Regarding interaction analysis, data were obtained by single-cycle kinetics analysis. Using Biacore T200 Evaluation Software, version 2.0, the obtained data were subjected to curve fitting in a bivalent analysis mode, and the following values were obtained: ka1 = 3.208E + 5, and kd1 = 3.461E - 7. Moreover, since evaluation can be carried out at K_{D} = kd1/ka1 in the bivalent analysis, the evaluation value K_{D} = kd1/ka1 = 3.461E - 7 / 3.208E + 5 = 1.078E - 12 was obtained. These results are shown in Fig. 5.

From the K_{D} value in the sensorgram shown in Fig. 5, it was confirmed that CEA-V2122 strongly binds to CEACAM5.

Furthermore, the interaction between CEA-V2122 and a modified biotin was also analyzed using Biacore T200. The modified biotin was specifically the title compound 14 described in Example 1 of International Publication WO2018/07239. In addition, the analysis method was specifically as follows. That is, an amine-coupling kit was used, a target value was set to be 5000 RU, and the purified CEA-V2122 was immobilized on Sensor Chip CM5. With regard to the concentrations of the analytes, 5 types of two-fold serial dilutions from 1E-08 M to 6.25E-10 M were used. Regarding interaction analysis, data were obtained by single-cycle kinetics analysis. Using Biacore T200 Evaluation Software, version 2.0, the obtained data were subjected to curve fitting in a bivalent analysis mode, and the following values were obtained: ka1 = 3.792E + 4, and kd1 = 4.424E - 6. Moreover, since evaluation can be carried out at K_{D} = kd1/ka1 in the bivalent analysis, the evaluation value K_{D} = kd1/ka1 = 3.792E + 4/4.424E - 6 = 1.167E - 10 was obtained. These results are shown in Fig. 6.

From the K_{D} value in the sensorgram shown in Fig. 6, it was confirmed that CEA-V2122 strongly binds to a modified biotin.

### Example 4: Cell staining of CEACAM5-expressing cell line using FITC-labeled CEA-V2122

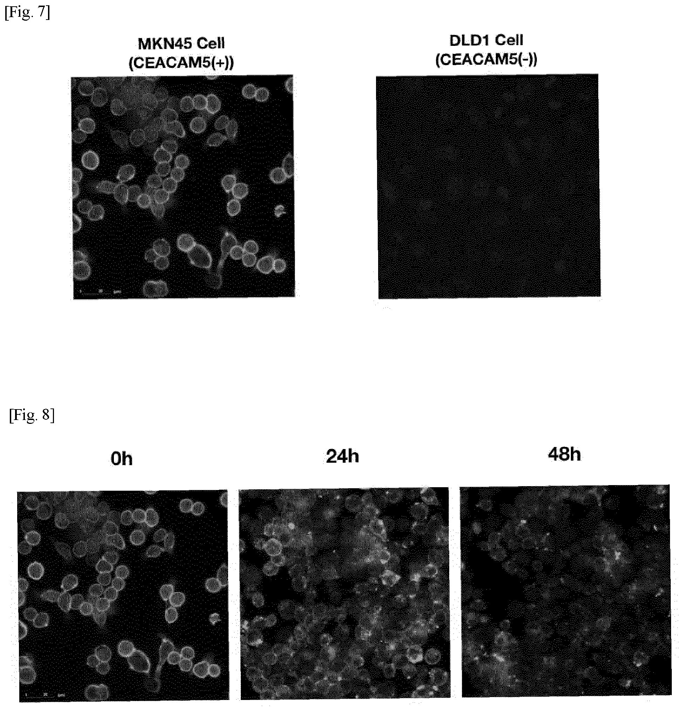

In order to stain a CEACAM5 expression-positive cancer cell line, FITC labeling was carried out on the cell line, using 100 µg of a purified CEA-V2122 protein. Specifically, labeling was carried out using Fluorescein Labeling Kit - NH₂ (DOJINDO LABORATORIES) in accordance with the protocol and amount described in the operating manual included with the kit, and the obtained product was defined to be CEA-V2122-FITC. Specific staining of the CEACAM5 expression-positive cancer cell line is as follows. That is, CEACAM5-positive human stomach cancer-derived MKN-45 cells and CEACAM5-negative human colon cancer-derived DLD1 cells were each seeded on a CELLSTAR, µClear, 96-well plate (Greiner) to a cell density of 2.0 x 10⁴ cells/well, and thereafter, the cells were cultured overnight. Subsequently, a culture solution containing 20 nM CEA-V2122-FITC and 1 µM Hoechist was added to the 96-well plate to a concentration of 100 µL/well, and the obtained mixture was then reacted at 4°C for 30 minutes. Thereafter, each image was taken using In Cell Analyzer 6000 (GE Healthcare Life Sciences). The results are shown in Fig. 7 and Fig. 8.

From the results shown in Fig. 7, it was confirmed that CEA-V2122-FITC specifically recognizes CEACAM5 on the surface of the cell membrane. In addition, from the results shown in Fig. 8, it was confirmed that after CEA-V2122-FITC has bound to CEACAM5, the CEACAM5 stays on the surface of the cell membrane.

### Example 5: In vitro cytotoxicity test using CEA-V2122 and photoactivatable compound-labeled modified biotins

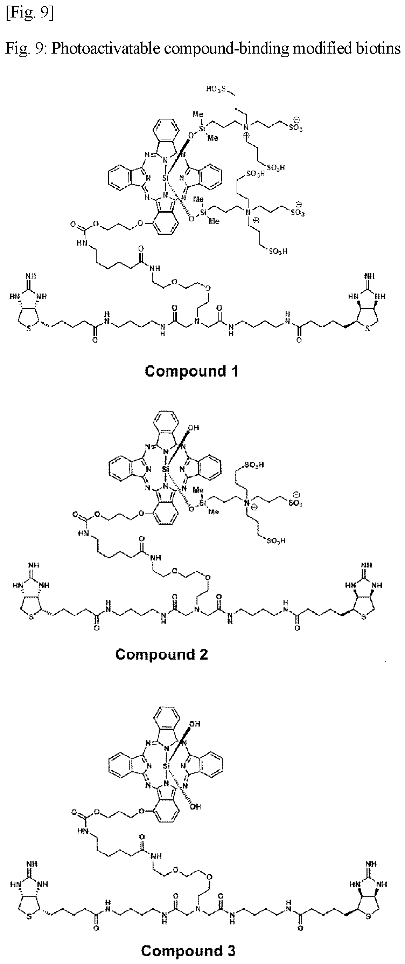

A cytotoxicity test was carried out using the photoactivatable compound-labeled modified biotins, namely, Compound 1, Compound 2, and Compound 3. These compounds are described in Japanese Patent Application No. 2018-149295. Compound 1, Compound 2, and Compound 3 are shown in Fig. 9. Specifically, MKN45 cells were seeded on a 96-well plate for cell culture, so that the cell count became 5 x 10³ cells/well and the amount of the culture solution became 50 µL/well, and thereafter, the cells were cultured overnight. A solution containing a complex of CEA-V2122 and a photoactivatable compound-labeled modified biotin was prepared, so that the molar ratio of CEA-V2122 and each compound became 1 : 2, and the solution was then incubated at room temperature for 10 minutes. Thereafter, the concentration of the reaction solution was adjusted with a culture solution, so that the final concentration of CEA-V2122 became 10 µg/mL. Regarding serial dilutions, 20 µg/mL was set to be an initial concentration, from 4-fold serial dilutions (5.0 µg/mL, 1.25 µg/mL, 0.312 µg/mL, and 0.078 µg/mL), 5 complex serial dilution solutions were prepared. Besides, a medium alone that contained no complex was used as a zero control.

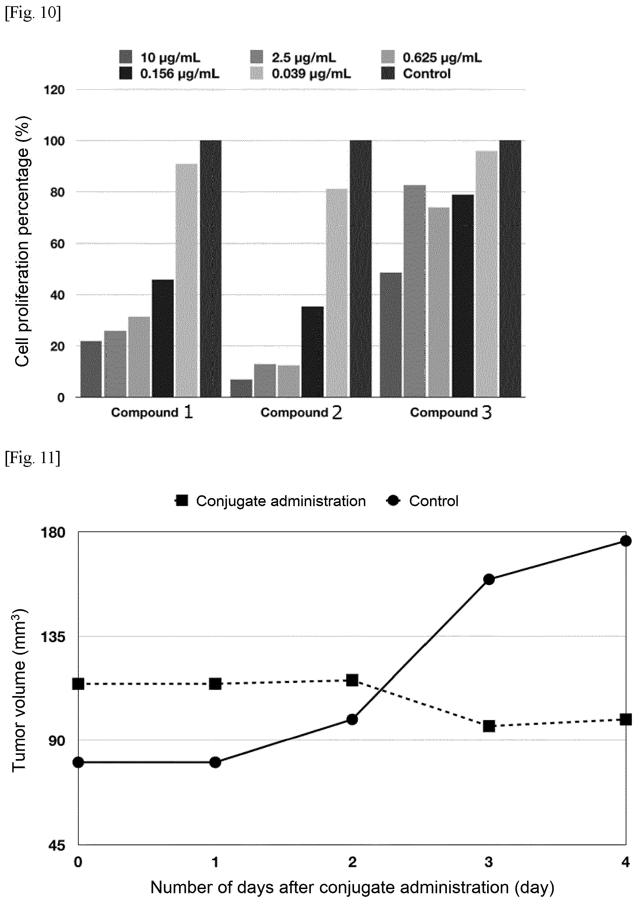

Such complex serial dilution solutions were each added in an amount of 50 µL/well to the cells which were cultured overnight, so that the final concentrations became 10 µg/mL, 2.5 µg/mL, 0.625 µg/mL, 0.156 µg/mL, and 0.039 µg/mL One hour and two hours after addition of the complex, the cells were irradiated with a light, using LED emitting a light having a wavelength of 690 ± 10 nm, so that the irradiation energy became 100 J/cm². Thereafter, the cells were cultured for 48 hours, and thereafter, a comparison was made in terms of the number of surviving cells, using Cell Counting Kit-8 (DOJINDO LABORATORIES). Each condition was set to be n = 3. The protocol and amount were determined in accordance with the instruction manuals included with the kit, and after addition of the reagent, the mixture was incubated for 1.5 hours at 37°C, in a CO₂ incubator. Thereafter, the absorbance at 450nm was measured, and the mean value was then calculated, followed by background collection. The control was set to be 100%, and the ratio of cell proliferation to the control under each condition was calculated. The results are shown in Fig. 10.

As shown in Fig. 10, it was confirmed that the complexes of CEA-V2122, and Compound 1, Compound 2 or Compound 3 exhibited cytotoxicity in a concentration-dependent manner.

### Example 6: In vivo cytotoxicity test using CEA-V2122 and photoactivatable compound-labeled modified biotin, in which xenograft mouse models are employed

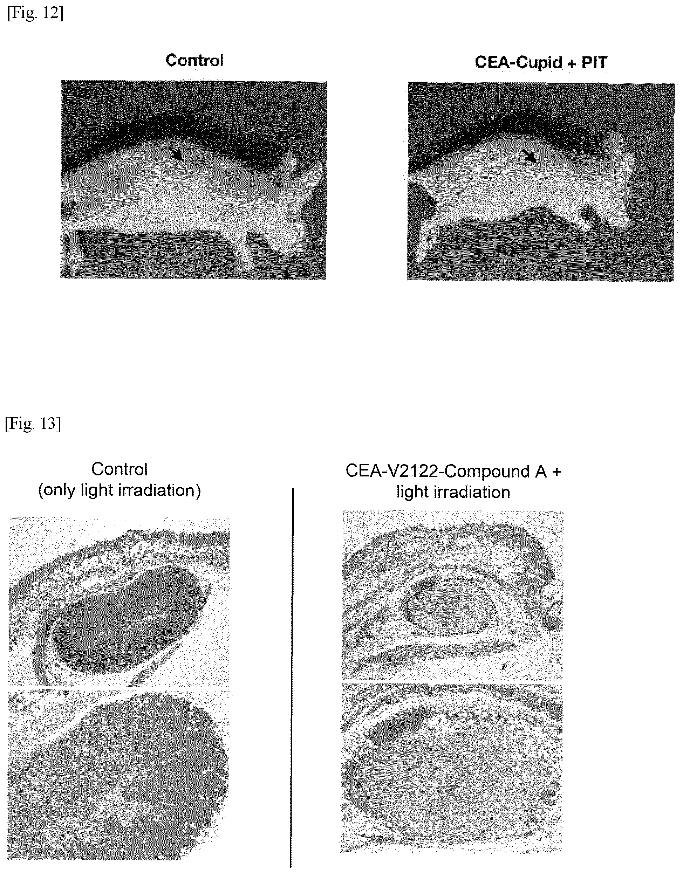

MKN45 cells were subcutaneously transplanted into nude mice to produce xenograft mouse models. That is, 4-week-old female nude mice were purchased, and the mice were then acclimated for 1 week. Thereafter, the MKN45 cells were transplanted in a cell count of 2 x 10⁵ cells per mouse into the subcutis thereof. Approximately 10 days after completion of the transplantation, 100 µg of a complex of CEA-V2122 and a photoactivatable compound-labeled modified biotin (Compound 1), as described in Example 4, was administered to the mice via the caudal vein thereof. Using an LED light source emitting a light having a wavelength of 690 nm (USHIO OPTO SMICONDUCTORS, INC., LD690D-66-60-550.), T-Cube LED Driver (THORLABS, NC0713145), and T-CUBE 15V POWER SUPPLY (THORLABS, TPS001), 6 hours after the administration, the mice were irradiated with the light having a wavelength of 690 nm, so that the irradiation energy became 230 J/cm². Twenty-four hours after administration of the complex, the mice were irradiated again with the light having a wavelength of 690 nm, so that the irradiation energy became 230 J/cm². A graph showing a change in the tumor volume is shown in Fig. 11. Photographs of the mice 5 days after administration of the complex are shown in Fig. 12. Individual mice were euthanized on the 7th day after administration of the complex and were then excised, and the tumor portions were subjected to pathological analysis. The specific method is as follows. That is, the subcutaneous tumor of each mouse and the peripheral tissues thereof were excised as a mass, and the mass was then immersed in a 4% paraformaldehyde solution (Wako Pure Chemical Industries, Ltd., 163-20145) at room temperature overnight, so that the tissues were fixed. The thus fixed subcutaneous tumor tissues were divided to a thickness of approximately 3 to 5 mm, so as to produce paraffin-embedded blocks. After that, using a microtome, sliced pathological specimens each having a thickness of 4 µm were produced.

Regarding hematoxylin and eosin staining, the sliced pathological specimen was immersed in a xylene solution (Wako Pure Chemical Industries, Ltd., 241-00091) at room temperature for 10 minutes to perform deparaffinization, and thereafter, hematoxylin staining (Sakura Finetek Japan Co., Ltd., #8650) and eosin staining (Sakura Finetek Japan Co., Ltd., #8660) were carried out. The results are shown in Fig. 13.

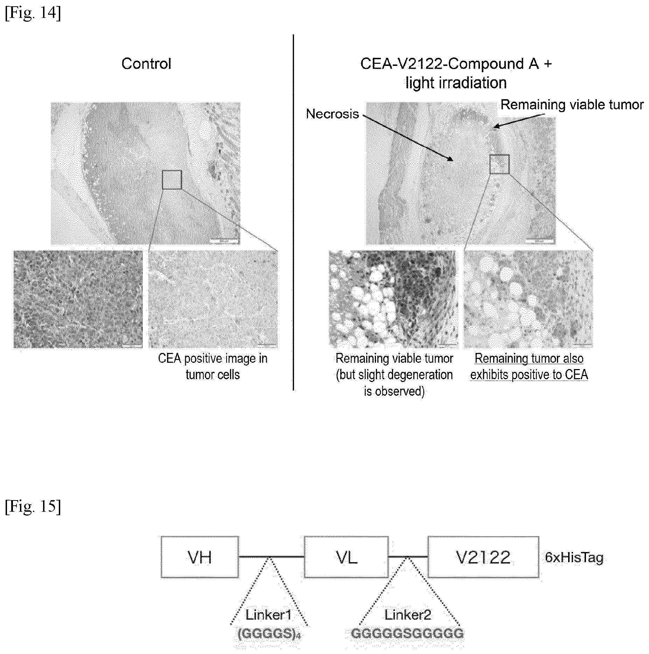

Moreover, immunostaining on CEACAM5 was carried out as follows. The sliced pathological specimen was immersed in a xylene solution (Wako Pure Chemical Industries, Ltd., 241-00091) at room temperature for 10 minutes to perform deparaffinization, and thereafter, the antigen was activated by performing an autoclave treatment (121°C, 5 minutes) using a citrate buffer (pH = 6.0). Thereafter, the resulting specimen was immersed in a 0.3% hydrogen peroxide (Wako Pure Chemical Industries, Ltd., 081-04215)/methanol (Wako Pure Chemical Industries, Ltd., 137-01823) solution at room temperature for 10 minutes to remove endogenous peroxidase. Non-specific reactions were blocked with a 2% BSA (Sigma Aldrich, A1470)/phosphate buffered saline solution. An anti-CEACAM5 antibody (R & D SYSTEMS, MAB41281, concentration: 1/100) was reacted with the specimen at 4°C overnight, and thereafter, immunostaining signals were visualized using Histostar (trademark) (MBL, #8460) and DAB Substrate Solution (MBL, #8469). Finally, nuclear staining was carried out using hematoxylin (Sakura Finetek Japan Co.,Ltd., #8650). The results are shown in Fig. 14.

From Fig. 13 and Fig. 14, it was confirmed that a complex of CEA-V2122 and Compound 1 is administered to a xenograft mouse model and the mouse model is then irradiated with a light with a wavelength of 690 nm, so that necrosis can be induced to the tumor of the xenograft mouse model.

### Example 7: Expression and purification of HER2-V2122 protein >

V2122 is a mutant streptavidin described in Example 3 of International Publication WO2015/125820 (SEQ ID NO: 4 shown in International Publication WO2015/125820). The amino acid sequence of V2122 is as set forth in SEQ ID NO: 12 in the sequence listing.

scFv-V2122 is prepared by binding a single-chain antibody (scFv) against HER2 (ERBB2) with the above-described V2122. This scFv-type anti-HER2 antibody is an scFv sequence described in Zhang H, et al., Therapeutic potential of an anti-HER2 single chain antibody-DM1 conjugates for the treatment of HER2-positive cancer. Signal Transduct Target Ther. 2017 May 19; 2: 17015. doi: 10.1038/sigtrans. 2017.15. The amino acid sequence of the scFv-type anti-HER2 antibody is as set forth in SEQ ID NO: 18 in the sequence listing. In addition, the structural drawing of HER2-V2122 prepared by binding the scFv-type anti-HER2 antibody with V2122 via an amino acid linker (GGGGGSGGGGG) (SEQ ID NO: 29) is shown in Fig. 15, and the amino acid sequence of HER2-V2122 is as set forth in SEQ ID NO: 19 in the sequence listing.

For the expression of a HER2-V2122 fusion protein, the DNA codon of a HER2-V2122 gene sequence, in which a pelB signal for secretion and expression in *Escherichia coli* had been incorporated into the N-terminus and a 6xHis-Tag sequence had been incorporated into the C-terminus, was optimized for *Escherichia coli*, thereby synthesizing an artificial gene. This amino acid sequence is as set forth in SEQ ID NO: 20 in the sequence listing, and the DNA sequence is as set forth in SEQ ID NO: 21 in the sequence listing.

As a specific protein expression vector, a vector prepared by incorporating a chaperone skp gene into MCS2 of a pETDuet1 vector was used. Regarding the skp gene, the DNA codon was optimized for *Escherichia coli* based on the amino acid sequence as set forth in SEQ ID NO: 6 in the sequence listing, thereby synthesizing an artificial gene. The synthesized skp gene was amplified by PCR, using the primers (AAGGAGATATACATATGGATAAAATTGCCATTGTTAATAT (SEQ ID NO: 23), and TTGAGATCTGCCATATGTTATTTCACTTGTTTCAGAACG (SEQ ID NO: 24)), and the amplified gene was then cloned into MCS2 of the pETDue1 vector linearized with the restriction enzyme NdeI, using In-Fusion HD Cloning Kit, so as to obtain pETDuet_skp. Subsequently, the HER2-V2122 gene was incorporated into MCS1 of pETDuet_skp. Specifically, the artificially synthesized HER2-V2122 gene was amplified by PCR, using the primers (AGAAGGAGATATACCATGAAATATCTGCTGCCGAC (SEQ ID NO: 25), and CGCCGAGCTCGAATTTTAATGATGGTGATGATGATG (SEQ ID NO: 26)). Moreover, pETDuet_skp was linearized by PCR, using the primers (GGTATATCTCCTTCTTAAAGTTAAAC (SEQ ID NO: 27), and AATTCGAGCTCGGCGCGCCTGCAG (SEQ ID NO: 28)). The HER2-V2122 amplified by PCR and the linearized pETDuet_skp were subjected to cloning using In-Fusion HD Cloning Kit. The cloned vector was confirmed by sequencing, in terms of the gene sequence incorporated therein, and thereafter, it was referred to as pETDuet_HER2-V2122_skp.

For the expression of the protein, pETDuet_HER2-V2122_skp was transformed into BL21(DE3) (Nippon Gene Co., Ltd.), which was then pre-cultured in 2xYT medium (SIGMA-ADLRICH) at 37°C overnight. The medium used in the pre-culture was added to a new medium for 100-fold dilution, and culture was then carried out at 37°C, until OD (600 nm) became 0.5 to 2.0. Subsequently, IPTG was added to the culture to a final concentration of 0.5 mM, and the obtained mixture was then cultured at 37°C for 4 hours. Thereafter, a culture supernatant was recovered and was then preserved at 4°C.

The HER2-V2122 protein was roughly purified according to a batch method utilizing a 6xHis-Tag added to the C-terminus. Specifically, cOmplete His-Tag Purification Resin equilibrated with buffer A (50 mM Tris-HCl, 0.2 M NaCl, 1 mM EDTA, and 5 mM Imidazole; pH 8.0) was added to the culture supernatant preserved at 4°C. The obtained mixture was stirred for 2 hours to overnight at 4° C, so that the protein was allowed to bind to the resin. Subsequently, the resin was recovered into a column, and a 20 column volume of washing operation was performed with buffer A. Thereafter, a roughly purified product of HER2-V2122 was recovered by elution with buffer B (50 mM Tris-HCl, 0.2 M NaCl, 1 mM EDTA, and 400 mM Imidazole; pH 8.0).

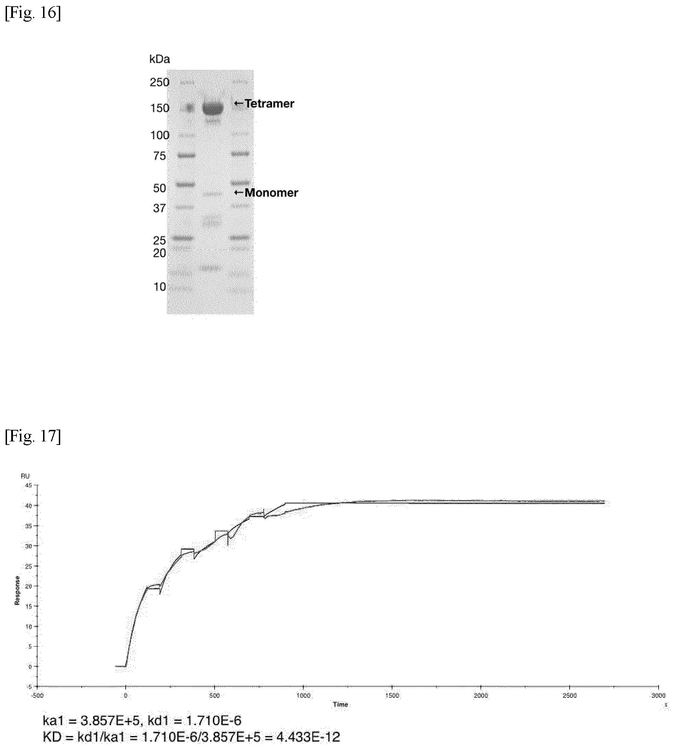

Subsequently, the roughly purified product was purified using a Protein L column. Specifically, 1 mL of Capto L (GE Healthcare) was filled into a PD-10 column and was then equilibrated with 10 column volume of PBS, and the aforementioned roughly purified product was then applied thereto. Thereafter, the resultant was washed with 10 column volume of PBS, was then eluted with 10 mM glycine hydrochloride (pH 2.0), and was then subjected to centrifugal concentration using Vivaspin Turbo 15 (MWCO 100,000). Moreover, using PD-10 (GE Healthcare), the buffer was replaced with PBS, and centrifugal concentration was further carried out using Vivaspin Turbo 4 (MWCO 100,000) to obtain a finally purified product. The purified product was subjected to CBB staining, and the purity of tetramer HER2-V2122 was assayed. The results are shown in Fig. 16. As an SDS-PAGE gel, Mini-PROTEAN TGX 4-15% (Bio-Rad) was used, and as a CBB staining solution, Bullet CBB Stain One (Ready To Use) (Nacalai Tesque, Inc.) was used.

From Fig. 16, it was confirmed that the purified HER2-V2122 comprises an approximately 150 kDa tetramer as a main component.

### Example 8: Evaluation of performance of HER2-V2122 by SPR

The affinity of HER2-V2122 for the antigen CEACAM5 was evaluated using a surface plasmon resonance (SPR) measuring device, Biacore T200 (GE Healthcare Life Sciences). Specifically, Recombinant Human ErbB2/Fc Chimera, Carrier Free (R & D SYSTEMS, 1129-ER-050) was immobilized on Sensor Chip CM5 (GE Healthcare Life Sciences) using an amine-coupling kit (GE Healthcare Life Sciences). The final amount of the ligand immobilized was 279 RU. Moreover, with regard to the purified HER2-V2122, two-fold serial dilutions from 1E-08 M to 6.25E-10 M were prepared as analytes. Regarding interaction analysis, data were obtained by single-cycle kinetics analysis. Using Biacore T200 Evaluation Software, version 2.0, the obtained data were subjected to curve fitting in a bivalent analysis mode, and the following values were obtained: ka1 = 3.857E + 5, and kd1 = 1.710E - 6. Moreover, since evaluation can be carried out at K_{D} = kd1/ka1 in the bivalent analysis, the evaluation value K_{D} = kd1/ka1 = 1.710E - 6 / 3.857E + 5 = 4.433E - 12 was obtained. These results are shown in Fig. 17.

From the sensorgram and the calculated K_{D} value shown in Fig. 17, it was confirmed that HER-V2122 recognizes ErbB2 and strongly binds thereto.

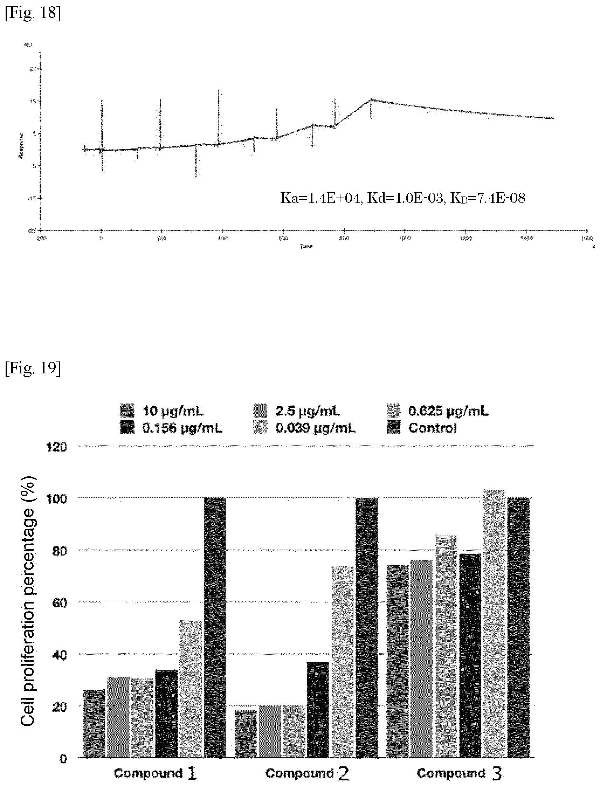

Furthermore, the interaction between HER2-V2122 and a modified biotin (Production Example 1, the compound represented by Formula 17) was also analyzed using Biacore T200. Specifically, an amine-coupling kit was used, a target value was set to be 5000 RU, and the purified CEA-V2122 was immobilized on Sensor Chip CM5. With regard to the concentrations of the analytes, 5 types of two-fold serial dilutions from 1E-08 M to 6.25E-10 M were used. Regarding interaction analysis, data were obtained by single-cycle kinetics analysis. Using Biacore T200 Evaluation Software, version 2.0, the obtained data were subjected to curve fitting in a bivalent analysis mode, and the following values were obtained: ka1 = 1.4E + 4, and kd1 = 1.0E - 3. Moreover, since evaluation can be carried out at K_{D} = kd1/ka1 in the bivalent analysis, the evaluation value K_{D} = kd1/ka1 = kd1 = 1.0E-3 / 1.4E + 4 = 7.4E - 8 was obtained. These results are shown in Fig. 18.

### Example 9: In vitro cytotoxicity test using HER2-V2122 and photoactivatable compound-labeled modified biotins

A cytotoxicity test was carried out using the photoactivatable compound-labeled modified biotins, namely, Compound 1, Compound 2, and Compound 3. These compounds are described in Japanese Patent Application No. 2018-149295. Compound 1, Compound 2, and Compound 3 are shown in Fig. 9. Specifically, SK-BR-3 cells cultured in a McCoy's medium supplemented with 10% FBS were seeded on a 96-well plate for cell culture, so that the cell count became 5 x 10³ cells/well and the amount of the culture solution became 50 µL/well, and thereafter, the cells were cultured overnight. A solution containing a complex of HER2-V2122 and a photoactivatable compound-labeled modified biotin was prepared, so that the molar ratio of HER2-V2122 and each compound became 1 : 2, and the solution was then incubated at room temperature for 10 minutes. Thereafter, the concentration of the reaction solution was adjusted with a culture solution, so that the final concentration of HER2-V2122 became 10 µg/mL. Regarding serial dilutions, 20 µg/mL was set to be an initial concentration, from 4-fold serial dilutions (5.0 µg/mL, 1.25 µg/mL, 0.312 µg/mL, and 0.078 µg/mL), 5 complex serial dilution solutions were prepared. Besides, a medium alone that contained no complex was used as a zero control.

Such complex serial dilution solutions were each added in an amount of 50 µL/well to the cells cultured overnight, so that the final concentrations became 10 µg/mL, 2.5 µg/mL, 0.625 µg/mL, 0.156 µg/mL, and 0.039 µg/mL. One hour and two hours after addition of the complex, the cells were irradiated with a light, using LED emitting a light having a wavelength of 690 ± 10 nm, so that the irradiation energy became 100 J/cm². Thereafter, the cells were cultured for 48 hours, and thereafter, a comparison was made in terms of the number of surviving cells, using Cell Counting Kit-8 (DOJINDO LABORATORIES). Each condition was set to be n = 3. The dosage and administration were determined in accordance with the instruction manuals included with the kit, and addition of the reagent, the mixture was incubated for 1.5 hours at 37°C, in a CO₂ incubator. Thereafter, the absorbance at 450nm was measured, and the mean value was then calculated, followed by background collection. The control was set to be 100%, and the ratio of cell proliferation to the control under each condition was calculated. The results are shown in Fig. 19.

As shown in Fig. 19, it was confirmed that the complex of HER2-Cupid and a photoactivatable compound-labeled modified biotin exhibits cytotoxicity in a concentration-dependent manner. 
SEQ ID NO: 12
AEAGITGTWSDQLGDTFIVTAGADGALTGTYENAVGGAESRYVLTGRYDSAPATDGSGTA
LGWTVAWKNNSKNAHSATTWSGQYVGGADAKINTQWLLTSGTTNANAWKSTLVGHDT
FTKVKPSAASHHHHHH
 
SEQ ID NO: 13 (sm3E-scFv sequence)

QVKLEQSGAEVVKPGASVKLSCKASGFNIKDSYMHWLRQGPGQRLEWIGWIDPENGDT

EYAPKFQGKATFTTDTSANTAYLGLSSLRPEDTAVYYCNEGTPTGPYYFDYWGQGTLVTV

SSGGGGSGGGGSGGGGSENVLTQSPSSMSVSVGDRVNIACSASSSVPYMHWLQQKPGKS
 
PKLLIYLTSNLASGVPSRFSGSGSGTDYSLTISSVQPEDAATYYCQQRSSYPLTFGGGTKLEI

K
 
SEQ ID NO: 14
QVKLEQSGAEVVKPGASVKLSCKASGFNIKDSYMHWLRQGPGQRLEWIGWIDPENGDT
EYAPKFQGKATFTTDTSANTAYLGLSSLRPEDTAVYYCNEGTPTGPYYFDYWGQGTLVTV
SSGGGGSGGGGSGGGGSENVLTQSPSSMSVSVGDRVNIACSASSSVPYMHWLQQKPGKS
PKLLIYLTSNLASGVPSRFSGSGSGTDYSLTISSVQPEDAATYYCQQRSSYPLTFGGGTKLEI
KGGGGSGGGGAEAGITGTWSDQLGDTFIVTAGADGALTGTYENAVGGAESRYVLTGRYD
SAPATDGSGTALGWTVAWKNNSKNAHSATTWSGQYVGGADAKINTQWLLTSGTTNANA
WKSTLVGHDTFTKVKPSAASHHHHHH
 
SEQ ID NO: 15

MKYLLPTAAAGLLLLAAQPAMAQVKLEQSGAEVVKPGASVKLSCKASGFNIKDSYMHW

LRQGPGQRLEWIGWIDPENGDTEYAPKFQGKATFTTDTSANTAYLGLSSLRPEDTAVYYC

NEGTPTGPYYFDYWGQGTLVTVSSGGGGSGGGGSGGGGSENVLTQSPSSMSVSVGDRVN

IACSASSSVPYMHWLQQKPGKSPKLLIYLTSNLASGVPSRFSGSGSGTDYSLTISSVQPEDA

ATYYCQQRSSYPLTFGGGTKLEIKGGGGSGGGGAEAGITGTWSDQLGDTFIVTAGADGAL

TGTYENAVGGAESRYVLTGRYDSAPATDGSGTALGWTVAWKNNSKNAHSATTWSGQYV

GGADAKINTQWLLTSGTTNANAWKSTLVGHDTFTKVKPSAASHHHHHH
 
SEQ ID NO: 16
ATGAAATATCTGCTGCCGACCGCAGCAGCGGGTCTGCTGCTGCTGGCAGCACAGCCTG
CAATGGCACAGGTTAAACTGGAACAGAGCGGTGCCGAAGTTGTTAAACCGGGTGCAA
GCGTTAAACTGAGCTGTAAAGCAAGCGGCTTTAACATCAAAGATAGCTATATGCATTGG
CTGCGTCAGGGTCCGGGTCAGCGTCTGGAATGGATTGGTTGGATTGATCCGGAAAATG
GTGATACCGAATATGCACCGAAATTTCAGGGTAAAGCAACCTTTACCACCGATACCAG
CGCAAATACCGCATATCTGGGTCTGAGCAGCCTGCGTCCGGAAGATACCGCAGTGTATT
ATTGTAATGAAGGCACCCCGACCGGTCCGTATTATTTCGATTATTGGGGTCAGGGCACC
 
CTGGTTACCGTTAGCAGCGGTGGTGGTGGTAGTGGTGGCGGTGGTTCAGGCGGTGGC
GGTAGCGAAAATGTTCTGACCCAGAGCCCGAGCAGCATGAGCGTTAGCGTTGGTGATC
GTGTTAATATTGCATGTAGCGCAAGCAGCAGCGTTCCGTACATGCACTGGCTGCAGCA
GAAACCGGGTAAAAGCCCGAAACTGCTGATTTATCTGACCAGCAATCTGGCAAGCGG
TGTTCCGAGCCGTTTTAGCGGTAGCGGTAGTGGCACCGATTATAGCCTGACCATTAGCA
GCGTGCAGCCTGAAGATGCAGCAACCTATTATTGTCAGCAGCGTAGCAGTTATCCGCT
GACCTTTGGTGGTGGCACCAAACTGGAAATTAAAGGGGGTGGTGGCTCAGGTGGCGG
AGGTGCAGAAGCAGGTATTACCGGTACATGGTCAGATCAGCTGGGTGATACCTTTATTG
TTACCGCAGGCGCAGATGGTGCACTGACCGGCACCTATGAAAATGCAGTTGGTGGTGC
AGAAAGCCGTTATGTGCTGACCGGTCGTTATGATAGCGCACCGGCAACCGATGGTAGC
GGCACCGCACTGGGTTGGACCGTTGCATGGAAAAATAACAGCAAAAATGCACATAGC
GCAACCACCTGGTCAGGTCAGTATGTGGGTGGTGCCGATGCCAAAATTAACACCCAGT
GGCTGCTGACCAGCGGTACAACCAATGCAAATGCCTGGAAAAGTACCCTGGTTGGTCA
TGATACATTCACCAAAGTTAAACCGAGCGCAGCAAGCCATCATCATCACCATCATTAA
 
SEQ ID NO: 17

MDKIAIVNMGSLFQQVAQKTGVSNTLENEFKGRASELQRMETDLQAKMKKLQSMKAGS

DRTKLEKDVMAQRQTFAQKAQAFEQDRARRSNEERGKLVTRIQTAVKSVANSQDIDLVV

DANAVAYNSSDVKDITADVLKQVK
 
SEQ ID NO: 18
EVQLVESGGGLVQPGGSLRLSCAASGFNIKDTYIHWVRQAPGKGLEWVARIYPTNGYTRY
ADSVKGRFTISADTSKNTAYLQMNSLRAEDTAVYYCSRWGGDGFYAMDYWGQGTLVTV
SSGGGGSGGGGSGGGGSGGGGSDIQMTQSPSSLSASVGDRVTITCRASQDVNTAVAWYQ
QKPGKAPKLLIYSASFLYSGVPSRFSGSRSGTDFTLTISSLQPEDFATYYCQQHYTTPPTFGQ
GTKVEIK
 
SEQ ID NO: 19
EVQLVESGGGLVQPGGSLRLSCAASGFNIKDTYIHWVRQAPGKGLEWVARIYPTNGYTRY
 
ADSVKGRFTISADTSKNTAYLQMNSLRAEDTAVYYCSRWGGDGFYAMDYWGQGTLVTV
SSGGGGSGGGGSGGGGSGGGGSDIQMTQSPSSLSASVGDRVTITCRASQDVNTAVAWYQ
QKPGKAPKLLIYSASFLYSGVPSRFSGSRSGTDFTLTISSLQPEDFATYYCQQHYTTPPTFGQ
GTKVEIKGGGGGSGGGGGAEAGITGTWSDQLGDTFIVTAGADGALTGTYENAVGGAESR
YVLTGRYDSAPATDGSGTALGWTVAWKNNSKNAHSATTWSGQYVGGADAKINTQWLLT
SGTTNANAWKSTLVGHDTFTKVKPSAASHHHHHH
 
SEQ ID NO: 20
MKYLLPTAAAGLLLLAAQPAMAEVQLVESGGGLVQPGGSLRLSCAASGFNIKDTYIHWV
RQAPGKGLEWVARIYPTNGYTRYADSVKGRFTISADTSKNTAYLQMNSLRAEDTAVYYCS
RWGGDGFYAMDYWGQGTLVTVSSGGGGSGGGGSGGGGSGGGGSDIQMTQSPSSLSASV
GDRVTITCRASQDVNTAVAWYQQKPGKAPKLLIYSASFLYSGVPSRFSGSRSGTDFTLTISS
LQPEDFATYYCQQHYTTPPTFGQGTKVEIKGGGGSGGGGAEAGITGTWSDQLGDTFIV
TAGADGALTGTYENAVGGAESRYVLTGRYDSAPATDGSGTALGWTVAWKNNSKNAHSAT
TWSGQYVGGADAKINTQWLLTSGTTNANAWKSTLVGHDTFTKVKPSAASHHHHHH
 
SEQ ID NO: 21
ATGAAATATCTGCTGCCGACCGCAGCAGCGGGTCTGCTGCTGCTGGCAGCACAGCCTG
CAATGGCAGAAGTTCAGCTGGTTGAAAGCGGTGGTGGTCTGGTTCAGCCTGGTGGTA
GCCTGCGTCTGAGCTGTGCAGCAAGCGGTTTTAACATTAAAGATACCTATATTCATTGG
GTGCGTCAGGCACCTGGTAAAGGTCTGGAATGGGTTGCACGTATTTATCCGACCAATG
GTTATACCCGTTATGCCGATAGCGTTAAAGGTCGTTTTACCATTAGCGCAGATACCAGC
AAAAATACCGCATACCTGCAGATGAATAGTCTGCGTGCAGAGGATACCGCAGTGTATTA
TTGTAGCCGTTGGGGTGGTGATGGTTTTTATGCAATGGATTATTGGGGTCAGGGCACCC
TGGTTACCGTTAGCTCAGGTGGAGGCGGTTCCGGTGGCGGAGGTTCCGGTGGAGGTG
GCTCCGGTGGCGGAGGTTCCGATATTCAGATGACCCAGAGTCCGAGCAGCCTGAGCG
CAAGCGTTGGTGATCGTGTGACCATTACCTGTCGTGCAAGCCAGGATGTTAATACAGC
AGTTGCATGGTATCAGCAGAAACCGGGTAAAGCACCGAAACTGCTGATTTATAGCGCA
AGCTTTCTGTATAGCGGTGTTCCGAGCCGTTTTAGCGGTAGCCGTAGCGGCACCGATTT
 
TACCCTGACCATTAGCAGCCTGCAGCCGGAAGATTTTGCAACCTATTATTGTCAGCAGC
ATTACACCACACCGCCTACCTTTGGCCAGGGCACCAAAGTTGAAATTAAAGGAGGTGG
CGGTGGATCCGGCGGAGGTGGCGGAGCAGAAGCAGGTATTACCGGTACATGGTCAGA
TCAGCTGGGTGATACCTTTATTGTTACCGCAGGCGCAGATGGTGCACTGACCGGCACCT
ATGAAAATGCAGTTGGTGGTGCAGAAAGCCGTTATGTGCTGACCGGTCGTTATGATAG
CGCACCGGCAACCGATGGTAGCGGCACCGCACTGGGTTGGACCGTTGCATGGAAAAA
TAACAGCAAAAATGCACATAGCGCAACCACCTGGTCAGGTCAGTATGTGGGTGGTGCC
GATGCCAAAATTAACACCCAGTGGCTGCTGACCAGCGGTACAACCAATGCAAATGCCT
GGAAAAGTACCCTGGTTGGTCATGATACATTCACCAAAGTTAAACCGAGCGCAGCAAG
CCATCATCATCACCATCATTAA
SEQ ID NO: 22
   GGGGSGGGG
SEQ ID NO: 23
   AAGGAGATATACATATGGATAAAATTGCCATTGTTAATAT
SEQ ID NO: 24
   TTGAGATCTGCCATATGTTATTTCACTTGTTTCAGAACG
SEQ ID NO: 25
   AGAAGGAGATATACCATGAAATATCTGCTGCCGAC
SEQ ID NO: 26
   CGCCGAGCTCGAATTTTAATGATGGTGATGATGATG
SEQ ID NO: 27
   GGTATATCTCCTTCTTAAAGTTAAAC
SEQ ID NO: 28
   AATTCGAGCTCGGCGCGCCTGCAG
SEQ ID NO: 29
   GGGGGSGGGGG

## Claims

1. A conjugate of a compound represented by the following formula (1) or a salt thereof and a phthalocyanine dye: 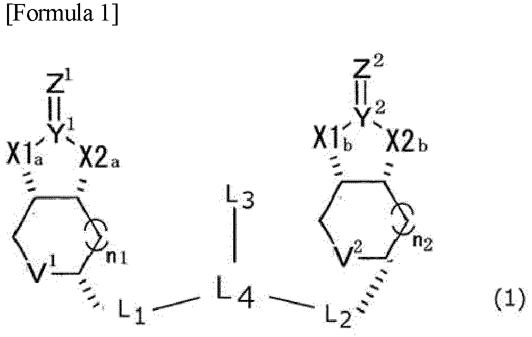 wherein
X1a, X1b, X2a and X2b each independently represent O or NH,
Y¹ and Y² each independently represent C or S,
Z¹ and Z² each independently represent O, S or NH,
V¹ and V² each independently represent S or S⁺-O⁻,
n1 and n2 each independently represent an integer of 0 or 1,
L₁ and L₂ each independently represent a divalent linking group,
L₃ represents a group comprising a functional group capable of binding to a phthalocyanine dye at the terminus, and
L₄ represents a trivalent linking group.

2. The conjugate according to claim 1, wherein the compound represented by the above formula (1) is represented by the following formula (2): 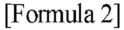 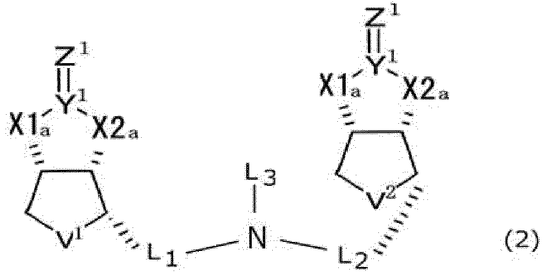 wherein each symbol is as defined in claim 1.

3. The conjugate according to claim 1 or 2, wherein X1a, X1b, X2a and X2b represent NH; Y¹ and Y² represent C; Z¹ and Z² represent NH; and V¹ and V² represent S.

4. The conjugate according to any one of claims 1 to 3, wherein L₁ and L₂ each independently represent a divalent linking group consisting of a combination of groups selected from -CONH-, -NHCO-, -COO-, -OCO-, -CO-, -O-, and an alkylene group containing 1 to 10 carbon atoms.

5. The conjugate according to any one of claims 1 to 4, wherein L₃ represents a group consisting of a combination of groups selected from -CONH-, -NHCO-, -COO-, -OCO-, -CO-, -O-, and an alkylene group containing 1 to 10 carbon atoms, and further comprising an amino group at the terminus.

6. The conjugate according to any one of claims 1 to 5, wherein the phthalocyanine dye is represented by the following formula (21): 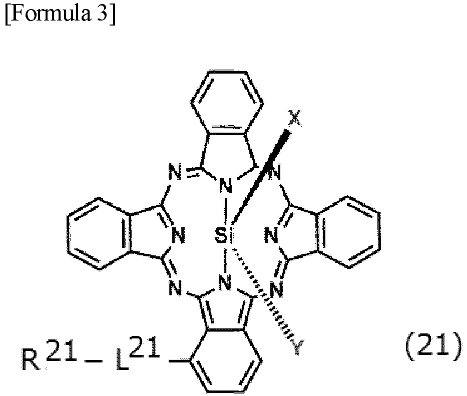 wherein L²¹ represents a divalent linking group; R²¹ represents a functional group capable of binding to the compound represented by the formula (1) or a salt thereof; and X and Y each independently represent a hydrophilic group, -OH, a hydrogen atom, or a substituent.

7. The conjugate according to claim 6, wherein the hydrophilic group represented by X and/or Y is the following:

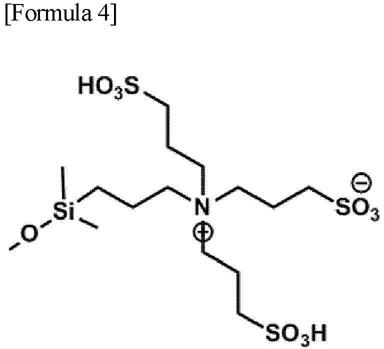

8. The conjugate according to claim 6 or 7, wherein L²¹ represents a group consisting of a combination of groups selected from -CONH-, -NHCO-, -COO-, -OCO-, -CO-, -O-, and an alkylene group containing 1 to 10 carbon atoms.

9. The conjugate according to any one of claims 6 to 8, wherein R²¹ represents an active ester group.

10. A conjugate represented by any one of the following:

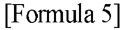

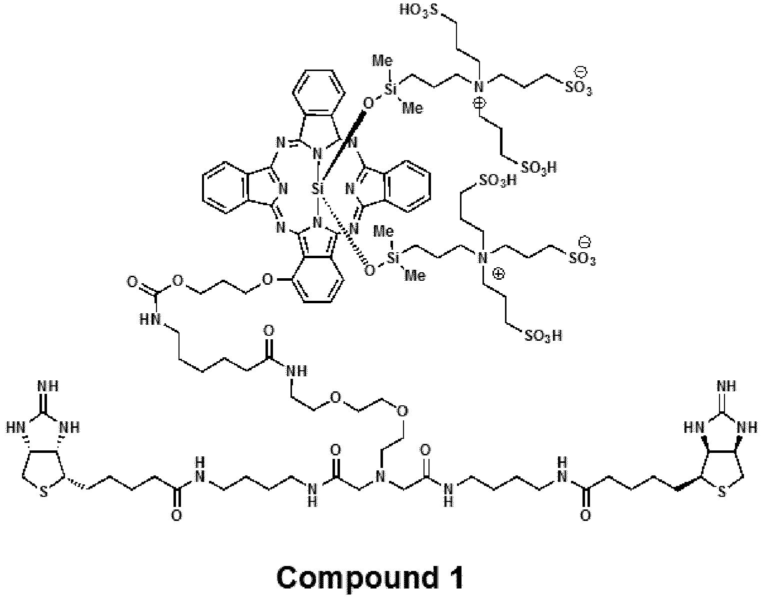

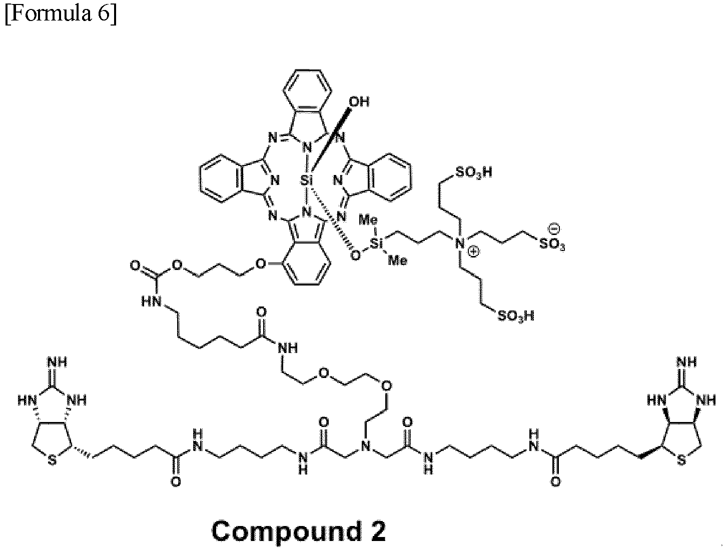

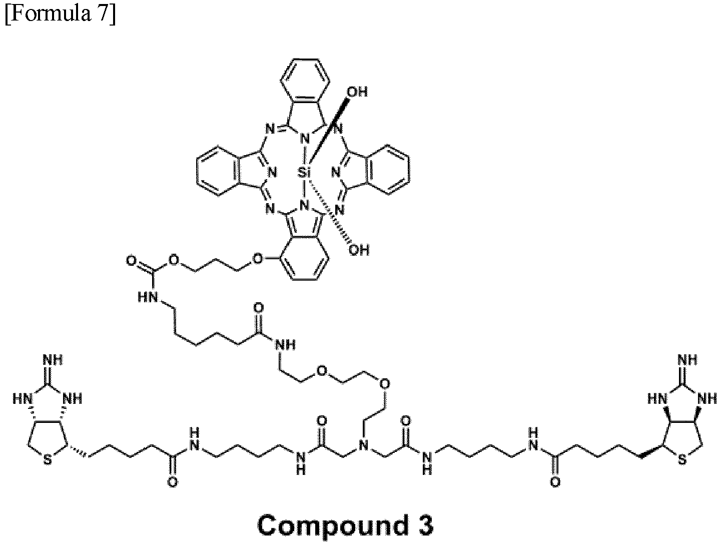

11. A therapeutic agent comprising the conjugate according to any one of claims 1 to 10.

12. A therapeutic kit, comprising: (1) the conjugate according to any one of claims 1 to 10; and (b) a conjugate of a mutant streptavidin comprising the amino acid sequence as set forth in SEQ ID NO: 1 and a molecular probe.

13. The therapeutic kit according to claim 12, wherein the molecular probe is an anti-EREG antibody, an anti-CEA antibody, or an anti-HER2 antibody.


SEQUENCE LISTING
<110>  The University of Tokyo
<110>  Savid Therapeutics Inc.
<120>  Conjugate of biotin-modified dimer and phthalocyanine dye
<130>  191247A
<160>  29
<210>  1
<211>  127
<212>  PRT
<213> Artificial Sequence
<220>
<223> Description of Artificial Sequence: Recombinant peptide
<400>  1
Ala Glu Ala Gly Ile Thr Gly Thr Trp Ser Asp Gln Leu Gly Asp Thr
1               5                   10                  15
Phe Ile Val Thr Ala Gly Ala Asp Gly Ala Leu Thr Gly Thr Tyr Glu
            20                  25                  30
Asn Ala Val Gly Gly Ala Glu Ser Arg Tyr Val Leu Thr Gly Arg Tyr
        35                  40                  45
Asp Ser Ala Pro Ala Thr Asp Gly Ser Gly Thr Ala Leu Gly Trp Thr
    50                  55                  60
Val Ala Trp Lys Asn Asn Ser Lys Asn Ala His Ser Ala Thr Thr Trp
65                  70                  75                  80
Ser Gly Gln Tyr Val Gly Gly Ala Asp Ala Lys Ile Asn Thr Gln Trp
                85                  90                  95
Leu Leu Thr Ser Gly Thr Thr Asn Ala Asn Ala Trp Lys Ser Thr Leu
            100                 105                 110
Val Gly His Asp Thr Phe Thr Lys Val Lys Pro Ser Ala Ala Ser
        115                 120                 125

<210>  2
<211>  1248
<212>  DNA
<213>  Recombinant
<220>
<221>  CDS
<222>  (1)..(1248)
<400>  2
atg aaa tac cta ttg cct acg gca gcc gct gga ttg tta tta ctc gcg      48
Met Lys Tyr Leu Leu Pro Thr Ala Ala Ala Gly Leu Leu Leu Leu Ala
1               5                   10                  15
gcc cag ccg gcc atg gcc cag gtg cag ctg cag cag tct ggc gcc gaa      96
Ala Gln Pro Ala Met Ala Gln Val Gln Leu Gln Gln Ser Gly Ala Glu
            20                  25                  30
gtg aag aaa cct ggc gcc tcc gtg aag gtg tcc tgc aag gcc tcc ggc      144
Val Lys Lys Pro Gly Ala Ser Val Lys Val Ser Cys Lys Ala Ser Gly
        35                  40                  45
ttc aac atc aag gac acc tac atg cac tgg gtg cga cag gcc cct gag      192
Phe Asn Ile Lys Asp Thr Tyr Met His Trp Val Arg Gln Ala Pro Glu
    50                  55                  60
cag ggc ctg gaa tgg atg ggc aga atc gac ccc ctg aac gac aag act      240
Gln Gly Leu Glu Trp Met Gly Arg Ile Asp Pro Leu Asn Asp Lys Thr
65                  70                  75                  80
aag tac gac ccc aag ttc cag ggc aga gtg acc atc acc gcc gac acc      288
Lys Tyr Asp Pro Lys Phe Gln Gly Arg Val Thr Ile Thr Ala Asp Thr
                85                  90                  95
tct acc aac acc gcc tac ctg gaa ctg tcc tcc ctg acc tct gag gac      336
Ser Thr Asn Thr Ala Tyr Leu Glu Leu Ser Ser Leu Thr Ser Glu Asp
            100                 105                 110
acc gcc gtg tac tac tgc gct aga ggc ggc gga gat ccc gtg ttc gtg      384
Thr Ala Val Tyr Tyr Cys Ala Arg Gly Gly Gly Asp Pro Val Phe Val
        115                 120                 125
tat tgg ggc cag ggc acc ctc gtg acc gtg tct gct tct tct ggc gga      432

Tyr Trp Gly Gln Gly Thr Leu Val Thr Val Ser Ala Ser Ser Gly Gly
    130                 135                 140
ggc gga tct ggg ggc gga ggt tct ggt ggt ggt gga agc ggt ggc ggt      480
Gly Gly Ser Gly Gly Gly Gly Ser Gly Gly Gly Gly Ser Gly Gly Gly
145                 150                 155                 160
gga tct ggc ggc gat atc cag atg acc cag tcc ccc agc tcc ctg tct      528
Gly Ser Gly Gly Asp Ile Gln Met Thr Gln Ser Pro Ser Ser Leu Ser
                165                 170                 175
gcc tct gtg ggc gac cgc gtg acc att aca tgc aag gcc agc cag gac      576
Ala Ser Val Gly Asp Arg Val Thr Ile Thr Cys Lys Ala Ser Gln Asp
            180                 185                 190
atc aac aag tac ctg gcc tgg tat cag cac aag ccc ggc cag gct cct      624
Ile Asn Lys Tyr Leu Ala Trp Tyr Gln His Lys Pro Gly Gln Ala Pro
        195                 200                 205
cgg ctg ctg atc cac tat acc tcc acc ctg cac ccc ggc atc cct tcc      672
Arg Leu Leu Ile His Tyr Thr Ser Thr Leu His Pro Gly Ile Pro Ser
    210                 215                 220
aga ttc tcc ggc tct ggc tcc ggc acc gac ttt acc ttc tcc atc tcc      720
Arg Phe Ser Gly Ser Gly Ser Gly Thr Asp Phe Thr Phe Ser Ile Ser
225                 230                 235                 240
agc ctg cag ccc gag gat atc gct acc tac tac tgc ctg cag tac gac      768
Ser Leu Gln Pro Glu Asp Ile Ala Thr Tyr Tyr Cys Leu Gln Tyr Asp
                245                 250                 255
aac ctg cgg acc ttc gga ggc ggc acc aag gtg gaa atc aag cgg acc      816
Asn Leu Arg Thr Phe Gly Gly Gly Thr Lys Val Glu Ile Lys Arg Thr
            260                 265                 270
agc ggc gga ggt gga agc ggt gga ggt gga gcc gaa gca ggt att acc      864
Ser Gly Gly Gly Gly Ser Gly Gly Gly Gly Ala Glu Ala Gly Ile Thr
        275                 280                 285
ggc acc tgg agc gat cag ctg ggc gat acc ttt att gtg acc gcc ggc      912
Gly Thr Trp Ser Asp Gln Leu Gly Asp Thr Phe Ile Val Thr Ala Gly
    290                 295                 300
gca gat ggt gcg ctg acc ggc acc tat gaa aat gcc gtg ggt ggt gcg      960
Ala Asp Gly Ala Leu Thr Gly Thr Tyr Glu Asn Ala Val Gly Gly Ala
305                 310                 315                 320
gaa agc cgt tat gtt ctg acc ggt cgt tat gat agc gca ccg gca acc     1008
Glu Ser Arg Tyr Val Leu Thr Gly Arg Tyr Asp Ser Ala Pro Ala Thr
                325                 330                 335
gat ggc agc ggc acc gcc ctg ggt tgg acc gtg gcg tgg aaa aac aat     1056
Asp Gly Ser Gly Thr Ala Leu Gly Trp Thr Val Ala Trp Lys Asn Asn
            340                 345                 350
agc aaa aac gcc cat agc gcg acc acc tgg agc ggc cag tat gtt ggc     1104
Ser Lys Asn Ala His Ser Ala Thr Thr Trp Ser Gly Gln Tyr Val Gly
        355                 360                 365
ggt gcc gat gcg aaa att aac acc cag tgg ctg ctg acc agc ggc acc     1152
Gly Ala Asp Ala Lys Ile Asn Thr Gln Trp Leu Leu Thr Ser Gly Thr
    370                 375                 380
acc aat gcc aat gcg tgg aaa agc acc ctg gtg ggt cat gat acc ttt     1200
Thr Asn Ala Asn Ala Trp Lys Ser Thr Leu Val Gly His Asp Thr Phe
385                 390                 395                 400
acc aaa gtt aaa ccg agc gcg gcc agc cac cac cac cac cac cac tga     1248
Thr Lys Val Lys Pro Ser Ala Ala Ser His His His His His His
                405                 410                 415
<210>  3
<211>  415
<212>  PRT
<213>  Recombinant
<400>  3
Met Lys Tyr Leu Leu Pro Thr Ala Ala Ala Gly Leu Leu Leu Leu Ala
1               5                   10                  15
Ala Gln Pro Ala Met Ala Gln Val Gln Leu Gln Gln Ser Gly Ala Glu
            20                  25                  30
Val Lys Lys Pro Gly Ala Ser Val Lys Val Ser Cys Lys Ala Ser Gly

35                  40                  45
Phe Asn Ile Lys Asp Thr Tyr Met His Trp Val Arg Gln Ala Pro Glu
    50                  55                  60
Gln Gly Leu Glu Trp Met Gly Arg Ile Asp Pro Leu Asn Asp Lys Thr
65                  70                  75                  80
Lys Tyr Asp Pro Lys Phe Gln Gly Arg Val Thr Ile Thr Ala Asp Thr
                85                  90                  95
Ser Thr Asn Thr Ala Tyr Leu Glu Leu Ser Ser Leu Thr Ser Glu Asp
            100                 105                 110
Thr Ala Val Tyr Tyr Cys Ala Arg Gly Gly Gly Asp Pro Val Phe Val
        115                 120                 125
Tyr Trp Gly Gln Gly Thr Leu Val Thr Val Ser Ala Ser Ser Gly Gly
    130                 135                 140
Gly Gly Ser Gly Gly Gly Gly Ser Gly Gly Gly Gly Ser Gly Gly Gly
145                 150                 155                 160
Gly Ser Gly Gly Asp Ile Gln Met Thr Gln Ser Pro Ser Ser Leu Ser
                165                 170                 175
Ala Ser Val Gly Asp Arg Val Thr Ile Thr Cys Lys Ala Ser Gln Asp
            180                 185                 190
Ile Asn Lys Tyr Leu Ala Trp Tyr Gln His Lys Pro Gly Gln Ala Pro
        195                 200                 205
Arg Leu Leu Ile His Tyr Thr Ser Thr Leu His Pro Gly Ile Pro Ser
    210                 215                 220
Arg Phe Ser Gly Ser Gly Ser Gly Thr Asp Phe Thr Phe Ser Ile Ser
225                 230                 235                 240
Ser Leu Gln Pro Glu Asp Ile Ala Thr Tyr Tyr Cys Leu Gln Tyr Asp
                245                 250                 255
Asn Leu Arg Thr Phe Gly Gly Gly Thr Lys Val Glu Ile Lys Arg Thr
            260                 265                 270
Ser Gly Gly Gly Gly Ser Gly Gly Gly Gly Ala Glu Ala Gly Ile Thr
        275                 280                 285
Gly Thr Trp Ser Asp Gln Leu Gly Asp Thr Phe Ile Val Thr Ala Gly
    290                 295                 300
Ala Asp Gly Ala Leu Thr Gly Thr Tyr Glu Asn Ala Val Gly Gly Ala
305                 310                 315                 320
Glu Ser Arg Tyr Val Leu Thr Gly Arg Tyr Asp Ser Ala Pro Ala Thr
                325                 330                 335
Asp Gly Ser Gly Thr Ala Leu Gly Trp Thr Val Ala Trp Lys Asn Asn
            340                 345                 350
Ser Lys Asn Ala His Ser Ala Thr Thr Trp Ser Gly Gln Tyr Val Gly
        355                 360                 365
Gly Ala Asp Ala Lys Ile Asn Thr Gln Trp Leu Leu Thr Ser Gly Thr
    370                 375                 380
Thr Asn Ala Asn Ala Trp Lys Ser Thr Leu Val Gly His Asp Thr Phe
385                 390                 395                 400
Thr Lys Val Lys Pro Ser Ala Ala Ser His His His His His His
                405                 410                 415
<210>  4
<211>  423
<212>  DNA
<213>  Recombinant
<220>
<221>  CDS
<222>  (1)..(423)
<400>  4
gat aaa att gcc att gtt aat atg ggt agc ctg ttt cag cag gtt gca       48
Asp Lys Ile Ala Ile Val Asn Met Gly Ser Leu Phe Gln Gln Val Ala
1               5                   10                  15
cag aaa acc ggt gtt agc aat acc ctg gaa aat gaa ttt aaa ggt cgt       96
Gln Lys Thr Gly Val Ser Asn Thr Leu Glu Asn Glu Phe Lys Gly Arg
            20                  25                  30
gca agc gaa ctg cag cgt atg gaa acc gat ctg cag gca aaa atg aaa      144
Ala Ser Glu Leu Gln Arg Met Glu Thr Asp Leu Gln Ala Lys Met Lys

35                  40                  45
aaa ctg cag agc atg aaa gca ggt agc gat cgt acc aaa ctg gaa aaa          192
Lys Leu Gln Ser Met Lys Ala Gly Ser Asp Arg Thr Lys Leu Glu Lys
    50                  55                  60
gat gtt atg gca cag cgt cag acc ttt gcc cag aaa gca cag gca ttt          240
Asp Val Met Ala Gln Arg Gln Thr Phe Ala Gln Lys Ala Gln Ala Phe
65                  70                  75                  80
gaa cag gat cgt gca cgt cgt agc aat gaa gaa cgt ggt aaa ctg gtt          288
Glu Gln Asp Arg Ala Arg Arg Ser Asn Glu Glu Arg Gly Lys Leu Val
                85                  90                  95
acc cgt att cag acc gca gtt aaa agc gtt gca aat agc cag gat att          336
Thr Arg Ile Gln Thr Ala Val Lys Ser Val Ala Asn Ser Gln Asp Ile
            100                 105                 110
gat ctg gtt gtt gat gca aat gcc gtt gcc tat aat agc agt gat gtg          384
Asp Leu Val Val Asp Ala Asn Ala Val Ala Tyr Asn Ser Ser Asp Val
        115                 120                 125
aaa gat att acc gca gac gtt ctg aaa caa gtg aaa taa                      423
Lys Asp Ile Thr Ala Asp Val Leu Lys Gln Val Lys
    130                 135                 140
<210>  5
<211>  140
<212>  PRT
<213>  Recombinant
<400>  5
Asp Lys Ile Ala Ile Val Asn Met Gly Ser Leu Phe Gln Gln Val Ala
1               5                   10                  15
Gln Lys Thr Gly Val Ser Asn Thr Leu Glu Asn Glu Phe Lys Gly Arg
            20                  25                  30
Ala Ser Glu Leu Gln Arg Met Glu Thr Asp Leu Gln Ala Lys Met Lys
        35                  40                  45
Lys Leu Gln Ser Met Lys Ala Gly Ser Asp Arg Thr Lys Leu Glu Lys
    50                  55                  60
Asp Val Met Ala Gln Arg Gln Thr Phe Ala Gln Lys Ala Gln Ala Phe
65                  70                  75                  80
Glu Gln Asp Arg Ala Arg Arg Ser Asn Glu Glu Arg Gly Lys Leu Val
                85                  90                  95
Thr Arg Ile Gln Thr Ala Val Lys Ser Val Ala Asn Ser Gln Asp Ile
            100                 105                 110
Asp Leu Val Val Asp Ala Asn Ala Val Ala Tyr Asn Ser Ser Asp Val
        115                 120                 125
Lys Asp Ile Thr Ala Asp Val Leu Lys Gln Val Lys
    130                 135                 140
<210>  6
<211>  29
<212>  DNA
<213> Artificial Sequence
<220>
<223> Description of Artificial Sequence: Synthetic DNA
<400>  6
tacatatgga taaaattgcc attgttaat                                          29
<210>  7
<211>  25
<212>  DNA
<213> Artificial Sequence
<220>
<223> Description of Artificial Sequence: Synthetic DNA
<400>  7
ttttatccat atgtatatct ccttc                                              25
<210>  8
<211>  20
<212>  DNA
<213> Artificial Sequence
<220>

<223> Description of Artificial Sequence: Synthetic DNA
<400> 8
ggtatatctc cttcttaaag 20
<210> 9
<211> 21
<212> DNA
<213> Artificial Sequence
<220>
<223> Description of Artificial Sequence: Synthetic DNA
<400> 9
aattcgagct cggcgcgcct g 21
<210> 10
<211> 35
<212> DNA
<213> Artificial Sequence
<220>
<223> Description of Artificial Sequence: Synthetic DNA
<400> 10
agaaggagat ataccatgaa atatctgctg ccgac 35
<210> 11
<211> 36
<212> DNA
<213> Artificial Sequence
<220>
<223> Description of Artificial Sequence: Synthetic DNA
<400> 11
cgccgagctc gaattttaat gatggtgatg atgatg 36
<210> 12
<211> 133
<212> PRT
<213> Artificial Sequence
<220>
<223> Description of Artificial Sequence: Recombinant protein
<400> 12
Ala Glu Ala Gly Ile Thr Gly Thr Trp Ser Asp Gln Leu Gly Asp Thr
1               5                   10                  15
Phe Ile Val Thr Ala Gly Ala Asp Gly Ala Leu Thr Gly Thr Tyr Glu
            20                  25                  30
Asn Ala Val Gly Gly Ala Glu Ser Arg Tyr Val Leu Thr Gly Arg Tyr
        35                  40                  45
Asp Ser Ala Pro Ala Thr Asp Gly Ser Gly Thr Ala Leu Gly Trp Thr
    50                  55                  60
Val Ala Trp Lys Asn Asn Ser Lys Asn Ala His Ser Ala Thr Thr Trp
65                  70                  75                  80
Ser Gly Gln Tyr Val Gly Gly Ala Asp Ala Lys Ile Asn Thr Gln Trp
                85                  90                  95
Leu Leu Thr Ser Gly Thr Thr Asn Ala Asn Ala Trp Lys Ser Thr Leu
            100                 105                 110
Val Gly His Asp Thr Phe Thr Lys Val Lys Pro Ser Ala Ala Ser His
        115                 120                 125
His His His His His
    130
<210> 13
<211> 241
<212> PRT
<213> Artificial Sequence
<220>
<223> Description of Artificial Sequence: Recombinant protein
<400> 13
Gln Val Lys Leu Glu Gln Ser Gly Ala Glu Val Val Lys Pro Gly Ala
1               5                   10                  15
Ser Val Lys Leu Ser Cys Lys Ala Ser Gly Phe Asn Ile Lys Asp Ser
            20                  25                  30

Tyr Met His Trp Leu Arg Gln Gly Pro Gly Gln Arg Leu Glu Trp Ile
        35                  40                  45
Gly Trp Ile Asp Pro Glu Asn Gly Asp Thr Glu Tyr Ala Pro Lys Phe
    50                  55                  60
Gln Gly Lys Ala Thr Phe Thr Thr Asp Thr Ser Ala Asn Thr Ala Tyr
65                  70                  75                  80
Leu Gly Leu Ser Ser Leu Arg Pro Glu Asp Thr Ala Val Tyr Tyr Cys
                85                  90                  95
Asn Glu Gly Thr Pro Thr Gly Pro Tyr Tyr Phe Asp Tyr Trp Gly Gln
            100                 105                 110
Gly Thr Leu Val Thr Val Ser Ser Gly Gly Gly Gly Ser Gly Gly Gly
        115                 120                 125
Gly Ser Gly Gly Gly Gly Ser Glu Asn Val Leu Thr Gln Ser Pro Ser
    130                 135                 140
Ser Met Ser Val Ser Val Gly Asp Arg Val Asn Ile Ala Cys Ser Ala
145                 150                 155                 160
Ser Ser Ser Val Pro Tyr Met His Trp Leu Gln Gln Lys Pro Gly Lys
                165                 170                 175
Ser Pro Lys Leu Leu Ile Tyr Leu Thr Ser Asn Leu Ala Ser Gly Val
            180                 185                 190
Pro Ser Arg Phe Ser Gly Ser Gly Ser Gly Thr Asp Tyr Ser Leu Thr
        195                 200                 205
Ile Ser Ser Val Gln Pro Glu Asp Ala Ala Thr Tyr Tyr Cys Gln Gln
    210                 215                 220
Arg Ser Ser Tyr Pro Leu Thr Phe Gly Gly Gly Thr Lys Leu Glu Ile
225                 230                 235                 240
Lys

<210>  14
<211>  383
<212>  PRT
<213> Artificial Sequence
<220>
<223> Description of Artificial Sequence: Recombinant protein
<400>  14
Gln Val Lys Leu Glu Gln Ser Gly Ala Glu Val Val Lys Pro Gly Ala
1               5                   10                  15
Ser Val Lys Leu Ser Cys Lys Ala Ser Gly Phe Asn Ile Lys Asp Ser
            20                  25                  30
Tyr Met His Trp Leu Arg Gln Gly Pro Gly Gln Arg Leu Glu Trp Ile
        35                  40                  45
Gly Trp Ile Asp Pro Glu Asn Gly Asp Thr Glu Tyr Ala Pro Lys Phe
    50                  55                  60
Gln Gly Lys Ala Thr Phe Thr Thr Asp Thr Ser Ala Asn Thr Ala Tyr
65                  70                  75                  80
Leu Gly Leu Ser Ser Leu Arg Pro Glu Asp Thr Ala Val Tyr Tyr Cys
                85                  90                  95
Asn Glu Gly Thr Pro Thr Gly Pro Tyr Tyr Phe Asp Tyr Trp Gly Gln
            100                 105                 110
Gly Thr Leu Val Thr Val Ser Ser Gly Gly Gly Gly Ser Gly Gly Gly
        115                 120                 125
Gly Ser Gly Gly Gly Gly Ser Glu Asn Val Leu Thr Gln Ser Pro Ser
    130                 135                 140
Ser Met Ser Val Ser Val Gly Asp Arg Val Asn Ile Ala Cys Ser Ala
145                 150                 155                 160
Ser Ser Ser Val Pro Tyr Met His Trp Leu Gln Gln Lys Pro Gly Lys
                165                 170                 175
Ser Pro Lys Leu Leu Ile Tyr Leu Thr Ser Asn Leu Ala Ser Gly Val
            180                 185                 190
Pro Ser Arg Phe Ser Gly Ser Gly Ser Gly Thr Asp Tyr Ser Leu Thr
        195                 200                 205
Ile Ser Ser Val Gln Pro Glu Asp Ala Ala Thr Tyr Tyr Cys Gln Gln
    210                 215                 220

Arg Ser Ser Tyr Pro Leu Thr Phe Gly Gly Gly Thr Lys Leu Glu Ile
225                 230                 235                 240
Lys Gly Gly Gly Gly Ser Gly Gly Gly Gly Ala Glu Ala Gly Ile Thr
                245                 250                 255
Gly Thr Trp Ser Asp Gln Leu Gly Asp Thr Phe Ile Val Thr Ala Gly
            260                 265                 270
Ala Asp Gly Ala Leu Thr Gly Thr Tyr Glu Asn Ala Val Gly Gly Ala
        275                 280                 285
Glu Ser Arg Tyr Val Leu Thr Gly Arg Tyr Asp Ser Ala Pro Ala Thr
    290                 295                 300
Asp Gly Ser Gly Thr Ala Leu Gly Trp Thr Val Ala Trp Lys Asn Asn
305                 310                 315                 320
Ser Lys Asn Ala His Ser Ala Thr Thr Trp Ser Gly Gln Tyr Val Gly
                325                 330                 335
Gly Ala Asp Ala Lys Ile Asn Thr Gln Trp Leu Leu Thr Ser Gly Thr
            340                 345                 350
Thr Asn Ala Asn Ala Trp Lys Ser Thr Leu Val Gly His Asp Thr Phe
        355                 360                 365
Thr Lys Val Lys Pro Ser Ala Ala Ser His His His His His His
    370                 375                 380
<210>  15
<211>  405
<212>  PRT
<213>  Artificial Sequence
<220>
<223>  Description of Artificial Sequence: Recombinant protein
<400>  15
Met Lys Tyr Leu Leu Pro Thr Ala Ala Ala Gly Leu Leu Leu Leu Ala
1               5                   10                  15
Ala Gln Pro Ala Met Ala Gln Val Lys Leu Glu Gln Ser Gly Ala Glu
            20                  25                  30
Val Val Lys Pro Gly Ala Ser Val Lys Leu Ser Cys Lys Ala Ser Gly
        35                  40                  45
Phe Asn Ile Lys Asp Ser Tyr Met His Trp Leu Arg Gln Gly Pro Gly
    50                  55                  60
Gln Arg Leu Glu Trp Ile Gly Trp Ile Asp Pro Glu Asn Gly Asp Thr
65                  70                  75                  80
Glu Tyr Ala Pro Lys Phe Gln Gly Lys Ala Thr Phe Thr Thr Asp Thr
                85                  90                  95
Ser Ala Asn Thr Ala Tyr Leu Gly Leu Ser Ser Leu Arg Pro Glu Asp
            100                 105                 110
Thr Ala Val Tyr Tyr Cys Asn Glu Gly Thr Pro Thr Gly Pro Tyr Tyr
        115                 120                 125
Phe Asp Tyr Trp Gly Gln Gly Thr Leu Val Thr Val Ser Ser Gly Gly
    130                 135                 140
Gly Gly Ser Gly Gly Gly Gly Ser Gly Gly Gly Gly Ser Glu Asn Val
145                 150                 155                 160
Leu Thr Gln Ser Pro Ser Ser Met Ser Val Ser Val Gly Asp Arg Val
                165                 170                 175
Asn Ile Ala Cys Ser Ala Ser Ser Ser Val Pro Tyr Met His Trp Leu
            180                 185                 190
Gln Gln Lys Pro Gly Lys Ser Pro Lys Leu Leu Ile Tyr Leu Thr Ser
        195                 200                 205
Asn Leu Ala Ser Gly Val Pro Ser Arg Phe Ser Gly Ser Gly Ser Gly
    210                 215                 220
Thr Asp Tyr Ser Leu Thr Ile Ser Ser Val Gln Pro Glu Asp Ala Ala
225                 230                 235                 240
Thr Tyr Tyr Cys Gln Gln Arg Ser Ser Tyr Pro Leu Thr Phe Gly Gly
                245                 250                 255
Gly Thr Lys Leu Glu Ile Lys Gly Gly Gly Gly Ser Gly Gly Gly Gly
            260                 265                 270
Ala Glu Ala Gly Ile Thr Gly Thr Trp Ser Asp Gln Leu Gly Asp Thr
        275                 280                 285

Phe Ile Val Thr Ala Gly Ala Asp Gly Ala Leu Thr Gly Thr Tyr Glu
    290                 295                 300
Asn Ala Val Gly Gly Ala Glu Ser Arg Tyr Val Leu Thr Gly Arg Tyr
305                 310                 315                 320
Asp Ser Ala Pro Ala Thr Asp Gly Ser Gly Thr Ala Leu Gly Trp Thr
                325                 330                 335
Val Ala Trp Lys Asn Asn Ser Lys Asn Ala His Ser Ala Thr Thr Trp
            340                 345                 350
Ser Gly Gln Tyr Val Gly Gly Ala Asp Ala Lys Ile Asn Thr Gln Trp
        355                 360                 365
Leu Leu Thr Ser Gly Thr Thr Asn Ala Asn Ala Trp Lys Ser Thr Leu
    370                 375                 380
Val Gly His Asp Thr Phe Thr Lys Val Lys Pro Ser Ala Ala Ser His
385                 390                 395                 400
His His His His His
                405
<210>  16
<211>  1218
<212>  DNA
<213> Artificial Sequence
<220>
<223> Description of Artificial Sequence: Recombinant DNA
<400>  16
atgaaatatc tgctgccgac cgcagcagcg ggtctgctgc tgctggcagc acagcctgca      60
atggcacagg ttaaactgga acagagcggt gccgaagttg ttaaaccggg tgcaagcgtt     120
aaactgagct gtaaagcaag cggctttaac atcaaagata gctatatgca ttggctgcgt     180
cagggtccgg gtcagcgtct ggaatggatt ggttggattg atccggaaaa tggtgatacc     240
gaatatgcac cgaaatttca gggtaaagca acctttacca ccgataccag cgcaaatacc     300
gcatatctgg gtctgagcag cctgcgtccg gaagataccg cagtgtatta ttgtaatgaa     360
ggcaccccga ccggtccgta ttatttcgat tattggggtc agggcaccct ggttaccgtt     420
agcagcggtg gtggtggtag tggtggcggt ggttcaggcg gtggcggtag cgaaaatgtt     480
ctgacccaga gcccgagcag catgagcgtt agcgttggtg atcgtgttaa tattgcatgt     540
agcgcaagca gcagcgttcc gtacatgcac tggctgcagc agaaaccggg taaaagcccg     600
aaactgctga tttatctgac cagcaatctg gcaagcggtg ttccgagccg ttttagcggt     660
agcggtagtg gcaccgatta tagcctgacc attagcagcg tgcagcctga agatgcagca     720
acctattatt gtcagcagcg tagcagttat ccgctgacct ttggtggtgg caccaaactg     780
gaaattaaag ggggtggtgg ctcaggtggc ggaggtgcag aagcaggtat taccggtaca     840
tggtcagatc agctgggtga tacctttatt gttaccgcag gcgcagatgg tgcactgacc     900
ggcacctatg aaaatgcagt tggtggtgca gaaagccgtt atgtgctgac cggtcgttat     960
gatagcgcac cggcaaccga tggtagcggc accgcactgg gttggaccgt tgcatggaaa    1020
aataacagca aaaatgcaca tagcgcaacc acctggtcag gtcagtatgt gggtggtgcc    1080
gatgccaaaa ttaacaccca gtggctgctg accagcggta caaccaatgc aaatgcctgg    1140
aaaagtaccc tggttggtca tgatacattc accaaagtta aaccgagcgc agcaagccat    1200
catcatcacc atcattaa                                                  1218
<210>  17
<211>  141
<212>  PRT
<213> Artificial Sequence
<220>
<223> Description of Artificial Sequence: Recombinant protein
<400>  17
Met Asp Lys Ile Ala Ile Val Asn Met Gly Ser Leu Phe Gln Gln Val
1               5                   10                  15
Ala Gln Lys Thr Gly Val Ser Asn Thr Leu Glu Asn Glu Phe Lys Gly
            20                  25                  30
Arg Ala Ser Glu Leu Gln Arg Met Glu Thr Asp Leu Gln Ala Lys Met
        35                  40                  45
Lys Lys Leu Gln Ser Met Lys Ala Gly Ser Asp Arg Thr Lys Leu Glu
    50                  55                  60
Lys Asp Val Met Ala Gln Arg Gln Thr Phe Ala Gln Lys Ala Gln Ala
65                  70                  75                  80
Phe Glu Gln Asp Arg Ala Arg Arg Ser Asn Glu Glu Arg Gly Lys Leu
                85                  90                  95

Val Thr Arg Ile Gln Thr Ala Val Lys Ser Val Ala Asn Ser Gln Asp
            100                 105                 110
Ile Asp Leu Val Val Asp Ala Asn Ala Val Ala Tyr Asn Ser Ser Asp
        115                 120                 125
Val Lys Asp Ile Thr Ala Asp Val Leu Lys Gln Val Lys
    130                 135                 140
<210> 18
<211> 247
<212> PRT
<213> Artificial Sequence
<220>
<223> Description of Artificial Sequence: Recombinant protein
<400> 18
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Asn Ile Lys Asp Thr
            20                  25                  30
Tyr Ile His Trp Val Arg Gln Ala Pro Gly Lys Gly Leu Glu Trp Val
        35                  40                  45
Ala Arg Ile Tyr Pro Thr Asn Gly Tyr Thr Arg Tyr Ala Asp Ser Val
    50                  55                  60
Lys Gly Arg Phe Thr Ile Ser Ala Asp Thr Ser Lys Asn Thr Ala Tyr
65                  70                  75                  80
Leu Gln Met Asn Ser Leu Arg Ala Glu Asp Thr Ala Val Tyr Tyr Cys
                85                  90                  95
Ser Arg Trp Gly Gly Asp Gly Phe Tyr Ala Met Asp Tyr Trp Gly Gln
            100                 105                 110
Gly Thr Leu Val Thr Val Ser Ser Gly Gly Gly Gly Ser Gly Gly Gly
        115                 120                 125
Gly Ser Gly Gly Gly Gly Ser Gly Gly Gly Gly Ser Asp Ile Gln Met
    130                 135                 140
Thr Gln Ser Pro Ser Ser Leu Ser Ala Ser Val Gly Asp Arg Val Thr
145                 150                 155                 160
Ile Thr Cys Arg Ala Ser Gln Asp Val Asn Thr Ala Val Ala Trp Tyr
                165                 170                 175
Gln Gln Lys Pro Gly Lys Ala Pro Lys Leu Leu Ile Tyr Ser Ala Ser
            180                 185                 190
Phe Leu Tyr Ser Gly Val Pro Ser Arg Phe Ser Gly Ser Arg Ser Gly
        195                 200                 205
Thr Asp Phe Thr Leu Thr Ile Ser Ser Leu Gln Pro Glu Asp Phe Ala
    210                 215                 220
Thr Tyr Tyr Cys Gln Gln His Tyr Thr Thr Pro Pro Thr Phe Gly Gln
225                 230                 235                 240
Gly Thr Lys Val Glu Ile Lys
                245
<210> 19
<211> 391
<212> PRT
<213> Artificial Sequence
<220>
<223> Description of Artificial Sequence: Recombinant protein
<400> 19
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Asn Ile Lys Asp Thr
            20                  25                  30
Tyr Ile His Trp Val Arg Gln Ala Pro Gly Lys Gly Leu Glu Trp Val
        35                  40                  45
Ala Arg Ile Tyr Pro Thr Asn Gly Tyr Thr Arg Tyr Ala Asp Ser Val
    50                  55                  60
Lys Gly Arg Phe Thr Ile Ser Ala Asp Thr Ser Lys Asn Thr Ala Tyr
65                  70                  75                  80
Leu Gln Met Asn Ser Leu Arg Ala Glu Asp Thr Ala Val Tyr Tyr Cys

                85                  90                  95
Ser Arg Trp Gly Gly Asp Gly Phe Tyr Ala Met Asp Tyr Trp Gly Gln
            100                 105                 110
Gly Thr Leu Val Thr Val Ser Ser Gly Gly Gly Gly Ser Gly Gly Gly
        115                 120                 125
Gly Ser Gly Gly Gly Gly Ser Gly Gly Gly Gly Ser Asp Ile Gln Met
    130                 135                 140
Thr Gln Ser Pro Ser Ser Leu Ser Ala Ser Val Gly Asp Arg Val Thr
145                 150                 155                 160
Ile Thr Cys Arg Ala Ser Gln Asp Val Asn Thr Ala Val Ala Trp Tyr
                165                 170                 175
Gln Gln Lys Pro Gly Lys Ala Pro Lys Leu Leu Ile Tyr Ser Ala Ser
            180                 185                 190
Phe Leu Tyr Ser Gly Val Pro Ser Arg Phe Ser Gly Ser Arg Ser Gly
        195                 200                 205
Thr Asp Phe Thr Leu Thr Ile Ser Ser Leu Gln Pro Glu Asp Phe Ala
    210                 215                 220
Thr Tyr Tyr Cys Gln Gln His Tyr Thr Thr Pro Pro Thr Phe Gly Gln
225                 230                 235                 240
Gly Thr Lys Val Glu Ile Lys Gly Gly Gly Gly Gly Ser Gly Gly Gly
                245                 250                 255
Gly Gly Ala Glu Ala Gly Ile Thr Gly Thr Trp Ser Asp Gln Leu Gly
            260                 265                 270
Asp Thr Phe Ile Val Thr Ala Gly Ala Asp Gly Ala Leu Thr Gly Thr
        275                 280                 285
Tyr Glu Asn Ala Val Gly Gly Ala Glu Ser Arg Tyr Val Leu Thr Gly
    290                 295                 300
Arg Tyr Asp Ser Ala Pro Ala Thr Asp Gly Ser Gly Thr Ala Leu Gly
305                 310                 315                 320
Trp Thr Val Ala Trp Lys Asn Asn Ser Lys Asn Ala His Ser Ala Thr
                325                 330                 335
Thr Trp Ser Gly Gln Tyr Val Gly Gly Ala Asp Ala Lys Ile Asn Thr
            340                 345                 350
Gln Trp Leu Leu Thr Ser Gly Thr Thr Asn Ala Asn Ala Trp Lys Ser
        355                 360                 365
Thr Leu Val Gly His Asp Thr Phe Thr Lys Val Lys Pro Ser Ala Ala
    370                 375                 380
Ser His His His His His His
385                 390
<210> 20
<211> 413
<212> PRT
<213> Artificial Sequence
<220>
<223> Description of Artificial Sequence: Recombinant protein
<400> 20
Met Lys Tyr Leu Leu Pro Thr Ala Ala Ala Gly Leu Leu Leu Leu Ala
1               5                   10                  15
Ala Gln Pro Ala Met Ala Glu Val Gln Leu Val Glu Ser Gly Gly Gly
            20                  25                  30
Leu Val Gln Pro Gly Gly Ser Leu Arg Leu Ser Cys Ala Ala Ser Gly
        35                  40                  45
Phe Asn Ile Lys Asp Thr Tyr Ile His Trp Val Arg Gln Ala Pro Gly
    50                  55                  60
Lys Gly Leu Glu Trp Val Ala Arg Ile Tyr Pro Thr Asn Gly Tyr Thr
65                  70                  75                  80
Arg Tyr Ala Asp Ser Val Lys Gly Arg Phe Thr Ile Ser Ala Asp Thr
                85                  90                  95
Ser Lys Asn Thr Ala Tyr Leu Gln Met Asn Ser Leu Arg Ala Glu Asp
            100                 105                 110
Thr Ala Val Tyr Tyr Cys Ser Arg Trp Gly Gly Asp Gly Phe Tyr Ala
        115                 120                 125
Met Asp Tyr Trp Gly Gln Gly Thr Leu Val Thr Val Ser Ser Gly Gly

130                 135                 140
Gly Gly Ser Gly Gly Gly Gly Ser Gly Gly Gly Gly Ser Gly Gly Gly
145                 150                 155                 160
Gly Ser Asp Ile Gln Met Thr Gln Ser Pro Ser Ser Leu Ser Ala Ser
                165                 170                 175
Val Gly Asp Arg Val Thr Ile Thr Cys Arg Ala Ser Gln Asp Val Asn
                180                 185                 190
Thr Ala Val Ala Trp Tyr Gln Gln Lys Pro Gly Lys Ala Pro Lys Leu
        195                 200                 205
Leu Ile Tyr Ser Ala Ser Phe Leu Tyr Ser Gly Val Pro Ser Arg Phe
    210                 215                 220
Ser Gly Ser Arg Ser Gly Thr Asp Phe Thr Leu Thr Ile Ser Ser Leu
225                 230                 235                 240
Gln Pro Glu Asp Phe Ala Thr Tyr Tyr Cys Gln Gln His Tyr Thr Thr
                245                 250                 255
Pro Pro Thr Phe Gly Gln Gly Thr Lys Val Glu Ile Lys Gly Gly Gly
                260                 265                 270
Gly Gly Ser Gly Gly Gly Gly Gly Ala Glu Ala Gly Ile Thr Gly Thr
        275                 280                 285
Trp Ser Asp Gln Leu Gly Asp Thr Phe Ile Val Thr Ala Gly Ala Asp
    290                 295                 300
Gly Ala Leu Thr Gly Thr Tyr Glu Asn Ala Val Gly Gly Ala Glu Ser
305                 310                 315                 320
Arg Tyr Val Leu Thr Gly Arg Tyr Asp Ser Ala Pro Ala Thr Asp Gly
                325                 330                 335
Ser Gly Thr Ala Leu Gly Trp Thr Val Ala Trp Lys Asn Asn Ser Lys
                340                 345                 350
Asn Ala His Ser Ala Thr Thr Trp Ser Gly Gln Tyr Val Gly Gly Ala
        355                 360                 365
Asp Ala Lys Ile Asn Thr Gln Trp Leu Leu Thr Ser Gly Thr Thr Asn
    370                 375                 380
Ala Asn Ala Trp Lys Ser Thr Leu Val Gly His Asp Thr Phe Thr Lys
385                 390                 395                 400
Val Lys Pro Ser Ala Ala Ser His His His His His His
                405                 410
<210>  21
<211>  1242
<212>  DNA
<213> Artificial Sequence
<220>
<223> Description of Artificial Sequence: Recombinant DNA
<400>  21
atgaaatatc tgctgccgac cgcagcagcg ggtctgctgc tgctggcagc acagcctgca      60
atggcagaag ttcagctggt tgaaagcggt ggtggtctgg ttcagcctgg tggtagcctg     120
cgtctgagct gtgcagcaag cggttttaac attaaagata cctatattca ttgggtgcgt     180
caggcacctg gtaaaggtct ggaatgggtt gcacgtattt atccgaccaa tggttatacc     240
cgttatgccg atagcgttaa aggtcgtttt accattagcg cagataccag caaaaatacc     300
gcatacctgc agatgaatag tctgcgtgca gaggataccg cagtgtatta ttgtagccgt     360
tggggtggtg atggttttta tgcaatggat tattggggtc agggcaccct ggttaccgtt     420
agctcaggtg gaggcggttc cggtggcgga ggttccggtg gaggtggctc cggtggcgga     480
ggttccgata ttcagatgac ccagagtccg agcagcctga gcgcaagcgt tggtgatcgt     540
gtgaccatta cctgtcgtgc aagccaggat gttaatacag cagttgcatg gtatcagcag     600
aaaccgggta aagcaccgaa actgctgatt tatagcgcaa gctttctgta tagcggtgtt     660
ccgagccgtt ttagcggtag ccgtagcggc accgatttta ccctgaccat tagcagcctg     720
cagccggaag attttgcaac ctattattgt cagcagcatt acaccacacc gcctaccttt     780
ggccagggca ccaaagttga aattaaagga ggtggcggtg gatccggcgg aggtggcgga     840
gcagaagcag gtattaccgg tacatggtca gatcagctgg gtgatacctt tattgttacc     900
gcaggcgcag atggtgcact gaccggcacc tatgaaaatg cagttggtgg tgcagaaagc     960
cgttatgtgc tgaccggtcg ttatgatagc gcaccggcaa ccgatggtag cggcaccgca    1020
ctgggttgga ccgttgcatg gaaaaataac agcaaaaatg cacatagcgc aaccacctgg    1080
tcaggtcagt atgtgggtgg tgccgatgcc aaaattaaca cccagtggct gctgaccagc    1140
ggtacaacca atgcaaatgc ctggaaaagt accctggttg gtcatgatac attcaccaaa    1200
gttaaaccga gcgcagcaag ccatcatcat caccatcatt aa                        1242

<210> 22
<211> 9
<212> PRT
<213> Artificial Sequence
<220>
<223> Description of Artificial Sequence: Recombinant linker
<400> 22
Gly Gly Gly Gly Ser Gly Gly Gly Gly
1               5
<210> 23
<211> 40
<212> DNA
<213> Artificial Sequence
<220>
<223> Description of Artificial Sequence: Synthesized DNA
<400> 23
aaggagatat acatatggat aaaattgcca ttgttaatat 40
<210> 24
<211> 39
<212> DNA
<213> Artificial Sequence
<220>
<223> Description of Artificial Sequence: Synthesized DNA
<400> 24
ttgagatctg ccatatgtta tttcacttgt ttcagaacg 39
<210> 25
<211> 35
<212> DNA
<213> Artificial Sequence
<220>
<223> Description of Artificial Sequence: Synthesized DNA
<400> 25
agaaggagat ataccatgaa atatctgctg ccgac 35
<210> 26
<211> 36
<212> DNA
<213> Artificial Sequence
<220>
<223> Description of Artificial Sequence: Synthesized DNA
<400> 26
cgccgagctc gaattttaat gatggtgatg atgatg 36
<210> 27
<211> 26
<212> DNA
<213> Artificial Sequence
<220>
<223> Description of Artificial Sequence: Synthesized DNA
<400> 27
ggtatatctc cttcttaaag ttaaac 26
<210> 28
<211> 24
<212> DNA
<213> Artificial Sequence
<220>
<223> Description of Artificial Sequence: Synthesized DNA
<400> 28
aattcgagct cggcgcgcct gcag 24
<210> 29
<211> 11
<212> PRT
<213> Artificial Sequence
<220>
<223> Description of Artificial Sequence: Recombinant linker

<400>  29
Gly Gly Gly Gly Gly Ser Gly Gly Gly Gly Gly
1               5                   10